# EUROPÄISCHE PATENTSCHRIFT

(11) **EP 4 212 134 B1**
(45) Veröffentlichungstag und Bekanntmachung des Hinweises auf die Patenterteilung: **21.08.2024**
(21) Anmeldenummer: 23150661.9
(22) Anmeldetag: 09.01.2023
(51) Int. Cl.: A61F 5/01, A61F 2/42

(54) **FUSSGELENK-ORTHESE**
ANKLE-FOOT ORTHOSIS
ORTHÈSE DE CHEVILLE

(30) Priorität: 17.01.2022 DE 102022100983
(43) Veröffentlichungstag der Anmeldung: 19.07.2023
(73) Patentinhaber: FXF GmbH, 92224 Amberg (DE)
(72) Erfinder: FISCHER, Franz, 92224 Amberg (DE)
(74) Vertreter: Benninger, Johannes

(56) Entgegenhaltungen:
- WO-A1-2018/019836
- DE-A1- 102015 207 584
- DE-U1- 202011 003 560

## Beschreibung

Die vorliegende Erfindung betrifft eine Fußgelenk-Orthese zur Begrenzung eines Bewegungsumfangs im Fußknöchelgelenk eines menschlichen Fußes und/oder zur äußeren mechanischen Stabilisierung des Bänderapparats im Fußknöchelgelenk.

Für die Therapie und Heilbehandlung von Traumata im Fuß und insbesondere im Bänderapparat des Fußgelenkes sind Orthesen in unterschiedlichen Ausführungen seit langem bekannt. Auch bei Lähmungserscheinungen im Fuß werden Orthesen eingesetzt, wie sie bspw. durch die DE 44 18 382 A1 beschrieben werden.

Doch auch bei weniger starken Einschränkungen, so etwa bei Fußfehlstellungen, können Orthesen eingesetzt werden, wie sie sich etwa durch die DE 199 22 625 A1 beschrieben finden.

Justierbare Orthesen zum Anschnallen an den Fuß für unterschiedlichste Anwendungen sind weiterhin durch die DE 299 09 113 U1 sowie durch die DE 20 2013 005 227 U1 offenbart.

Die DE 10 2006 040 783 A1 offenbart eine Orthese zur Korrektur von Gelenkfehlstellungen im oberen und unteren Sprunggelenk.

Eine Knöchel-Fuß-Orthese mit einer beweglichen Feder findet sich in der DE 10 2012 011 466 A1 beschrieben.

Gemeinsames Merkmal der bekannten Orthesen ist ihre relativ voluminöse Bauart, die es schwierig erscheinen lässt, diese mit herkömmlichem Schuhwerk zu kombinieren.

Darüber hinaus ist eine Testorthese zur Unterstützung einer Fußhebe- und Fußsenkbewegung aus der DE 20 2011 003 560 U1 bekannt, die den Oberbegriff des Anspruchs 1 offenbart. Ein Unterschenkel des zu unterstützenden Fußes wird von einer Unterschenkelfassung umgriffen, während ein sandalenartiger Fußteil, der mit der Unterschenkelfassung über ein Gelenk verbunden ist, den Fuß aufnimmt. Weiterhin ist ein Aktuator vorgesehen, der mit der Unterschenkelfassung und dem Fußteil verbunden ist und der die Fußhebe- und die Fußsenkbewegungen unterstützt. Durch Betätigung eines mit dem Aktuator verbundenen Umschalters kann der Aktuator veranlasst werden, entweder die Fußhebebewegung oder die Fußsenkbewegung zu unterstützen. Bei dem Aktuator kann es sich um einen Elektromotor oder um eine Feder handeln, so dass es sich um eine fremdkraftunterstützte Orthese handelt.

Demgegenüber kann es als ein vorrangiges Ziel der vorliegenden Erfindung betrachtet werden kann, eine möglichst kompakt bauende und universell einsetzbare Fußgelenk-Orthese anzubieten, die keine Fremdkraftunterstützung benötigt und die sich vorzugsweise unterhalb einer herkömmlichen Fußoberbekleidung tragen und/oder mit einem Schuh oder Stiefel soweit kombinieren lässt, dass sie äußerlich kaum wahrgenommen wird.

Dieses Ziel der Erfindung wird mit dem Gegenstand des unabhängigen Anspruchs erreicht. Merkmale vorteilhafter Weiterbildungen der Erfindung finden sich in den abhängigen Ansprüchen.

So schlägt die Erfindung zur Erreichung des genannten Ziels eine Fußgelenk-Orthese mit den Merkmalen des unabhängigen Anspruchs vor, die in erster Linie der Begrenzung eines Bewegungsumfangs im Fußknöchelgelenk eines menschlichen Fußes und/oder zur mechanischen Stabilisierung des Bänderapparats im Fußknöchelgelenk dient. Diese erfindungsgemäße Orthese umfasst mindestens zwei gelenkig miteinander verbundene Teilabschnitte.

Dabei ist ein unterer Teilabschnitt einer Lateralseite des zu stabilisierenden Fußes zuordenbar, während ein oberer Teilabschnitt einer Lateralseite des Unterschenkels oberhalb des Knöchelgelenks des Fußes zuordenbar ist.

Weiterhin ist erfindungsgemäß vorgesehen, dass der obere Teilabschnitt und der untere Teilabschnitt mittels eines in Nähe des Knöchelgelenks befindlichen, spielbehafteten Gelenks beweglich miteinander verbunden sind.

Außerdem ist proximal beabstandet zu diesem Gelenk ein Führungselement zur Winkel- und/oder Wegbegrenzung der Beweglichkeit und Verschwenkbarkeit der oberen und unteren Teilabschnitte gegeneinander angeordnet. Das Führungselement sorgt in Zusammenwirkung mit dem Gelenk dafür, dass jeweils die maximalen Schwenkwinkel sowohl bei einer Dorsalextension als auch bei einer Plantarflexion des Fußes auf definierte Grenzwinkel einschränkt bzw. begrenzt sind.

Damit kann gemeint sein, dass das Führungselement in Zusammenwirkung mit dem Gelenk dafür sorgen oder verantwortlich sein kann, dass im Wesentlichen nur die maximalen Schwenkwinkel sowohl bei einer Dorsalextension als auch bei einer Plantarflexion des Fußes auf definierte Grenzwinkel einschränkt bzw. begrenzt sind, während andere Bewegungsrichtungen des Fußes entweder nicht oder nur in minimalem Ausmaß zugelassen werden. Damit kann aber auch gemeint sein, dass über andere Bewegungsrichtungen des Fußes noch keine Aussage getroffen werden soll.

Wahlweise oder zusätzlich kann das Führungselement in Zusammenwirkung mit dem Gelenk dafür sorgen, dass ein Supinationswinkel sowie ein Pronationswinkel im Fußgelenk durch Einschränkung oder Begrenzung der Anstellwinkel zwischen oberem und unterem Teilabschnitt begrenzt sind. Damit ist gemeint, dass das Führungselement in Zusammenwirkung mit dem Gelenk dafür sorgen oder verantwortlich sein kann, dass nicht nur die maximalen Schwenkwinkel bei einer Dorsalextension und bei einer Plantarflexion des Fußes definiert sind, sondern dass das Führungselement in Zusammenwirkung mit dem Gelenk darüber hinaus auch dafür sorgt, einen maximal zulässigen Supinationswinkel und einen maximal zulässigen Pronationswinkel im Fußgelenk zu definieren, während wiederum über weitere Bewegungsrichtungen keine Aussage getroffen sein soll.

Wenn hier davon gesprochen wird, dass der untere Teilabschnitt der Lateralseite des zu stabilisierenden Fußes zuordenbar ist, so kann dies einerseits eine äußere Fixierung und/oder Montage an der Außenseite des Fußes bedeuten. Diese Definition soll jedoch gleichermaßen eine Eingriffsmöglichkeit in den menschlichen Organismus umfassen, etwa durch eine Montage des unteren Teilabschnittes am Knochenapparat des betroffenen Menschen mittels eines operativen und chirurgischen Eingriffes. Eine solche Montage am Knochenapparat kann bspw. durch Verkleben, durch Verschrauben, durch Verklammern oder durch Einfügen einer mehrteiligen Draht- und/oder Seilstruktur erfolgen, wobei eine solche mehrteilige Draht- und/oder Seilstruktur wiederum verklebt, verschraubt, verknotet, verklammert oder auf sonstige Weise mit unterschiedlichen, sinnvollerweise voneinander beabstandeten Knochenteilen verbunden werden kann.

Im Ergebnis wird damit eine stabile Verankerung des unteren Teilabschnittes an der Lateralseite des zu stabilisierenden Fußes - ob außerhalb des Körpers oder subkutan bzw. einzelne oder mehrere Fußknochen miteinbeziehend - geschaffen, die ein definiertes Widerlager für das spielbehaftete Gelenk liefern kann.

Wenn zudem an dieser Stelle davon gesprochen wird, dass der obere Teilabschnitt der Lateralseite des Unterschenkels oberhalb des Knöchelgelenks des zu stabilisierenden Fußes zuordenbar ist, so kann dies einerseits eine äußere Fixierung und/oder Montage an der Außenseite des Unterschenkels bedeuten. Diese Definition soll jedoch gleichermaßen eine Eingriffsmöglichkeit in den menschlichen Organismus umfassen, etwa durch eine Montage des oberen Teilabschnittes am Knochenapparat des Unterschenkels des betroffenen Menschen mittels eines operativen und chirurgischen Eingriffes. Eine solche Montage am Knochenapparat kann bspw. durch Verkleben, durch Verschrauben, durch Verklammern oder durch Einfügen einer mehrteiligen Draht- und/oder Seilstruktur erfolgen, wobei eine solche mehrteilige Draht- und/oder Seilstruktur wiederum verklebt, verschraubt, verknotet, verklammert oder auf sonstige Weise mit unterschiedlichen, sinnvollerweise voneinander beabstandeten Knochenabschnitten des Schienbeins und/oder des Wadenbeins verbunden werden kann.

Im Ergebnis wird damit eine stabile Verankerung des oberen Teilabschnittes an der Lateralseite des Unterschenkels oberhalb des zu stabilisierenden Fußes - ob außerhalb des Körpers oder subkutan bzw. einzelne oder mehrere Unterschenkelknochen miteinbeziehend - geschaffen, die ein definiertes Widerlager für das spielbehaftete Gelenk liefern kann.

Beide Varianten können wahlweise in beliebiger Weise miteinander kombiniert werden, so etwa eine Verankerung des unteren Teilabschnittes sowie des oberen Teilabschnittes am jeweiligen Knochenapparat, so dass hierbei sinnvollerweise auch das spielbehaftete Gelenk unterhalb der Haut platziert wird. Wahlweise kann das spielbehaftete Gelenk jedoch aus Platzgründen teilweise oder vollständig außerhalb des menschlichen Organismus positioniert sein, so dass gelenknahe Abschnitte des unteren sowie des oberen Teilabschnittes jeweils die Haut durchdringen können.

Wahlweise kommt auch eine Verankerung des unteren Teilabschnittes am jeweiligen Knochenapparat in Frage, während der obere Teilabschnitt ohne operative oder chirurgische Eingriffe an der Außenseite des Unterschenkels angelegt oder fixiert werden kann, entweder temporär oder dauerhaft. Sinnvollerweise wird bei dieser Variante auch das spielbehaftete Gelenk außerhalb der Haut platziert, so dass lediglich ein gelenknaher Abschnitt des unteren Teilabschnittes die Haut durchdringt.

Ebenso denkbar ist eine weitere Variante, bei der lediglich der obere Teilabschnitt am jeweiligen Knochenapparat des betroffenen Unterschenkels verankert wird, während der untere Teilabschnitt ohne operative oder chirurgische Eingriffe an der Außenseite des Fußes angelegt oder fixiert werden kann, entweder temporär oder dauerhaft. Sinnvollerweise wird bei dieser Variante auch das spielbehaftete Gelenk außerhalb der Haut platziert, so dass lediglich ein gelenknaher Abschnitt des oberen Teilabschnittes die Haut zu durchdringen hat.

Da sich das Führungselement in unmittelbarer Nähe zum spielbehafteten Gelenk befindet, ist im Einzelfall zu entscheiden, ob bei einer Montage des oberen und/oder des unteren Teilabschnittes mittels operativen oder chirurgischen Eingriffes am jeweiligen Knochenapparat auch das Führungselement gemeinsam mit dem Gelenk (teilweise oder abschnittsweise) unterhalb der Haut oder (teilweise oder abschnittsweise) außerhalb des betroffenen menschlichen Organismus platziert werden kann.

Wahlweise bzw. gemäß einer Ausführungsvariante der erfindungsgemäßen Fußgelenk-Orthese ist der untere Teilabschnitt an der Lateralseite des zu stabilisierenden Fußes anlegbar und/oder dort fixierbar. Damit kann etwa eine Fixierung mittels Bandes, mittels mehrerer Bänder, mittels einer am Fuß fixierbaren und/oder den Fuß zumindest teilweise umschlingenden Netzstruktur o. dgl. gemeint sein. Denkbar sind jedoch auch temporäre Fixierungen durch Verklebungen an der Haut, wobei diese Verklebungen entweder nach einiger Zeit selbsttätig und allmählich an Haftkraft verlieren und somit nach gewisser Tragezeit der Orthese bzw. des unteren Teilabschnittes der Orthese wieder gelöst werden können. Stattdessen sind jedoch auch Verklebungen denkbar, die dadurch wieder von der Haut gelöst werden können, indem die Klebestellen mit geeigneten Lösungsmitteln benetzt und beaufschlagt werden.

Wahlweise bzw. gemäß einer Ausführungsvariante der erfindungsgemäßen Fußgelenk-Orthese ist der obere Teilabschnitt an der Lateralseite des Unterschenkels oberhalb des Knöchelgelenks des Fußes anlegbar und/oder dort fixierbar. Damit kann etwa eine Fixierung mittels Bandes, mittels mehrerer Bänder, mittels einer am Unterschenkel fixierbaren und/oder den Unterschenkel zumindest teilweise umschlingenden Netzstruktur o. dgl. gemeint sein. Denkbar sind jedoch auch temporäre Fixierungen durch Verklebungen an der Haut, wobei diese Verklebungen entweder nach einiger Zeit selbsttätig und allmählich an Haftkraft verlieren und somit nach gewisser Tragezeit der Orthese bzw. des oberen Teilabschnittes der Orthese wieder gelöst werden können. Stattdessen sind jedoch auch Verklebungen denkbar, die dadurch wieder von der Haut gelöst werden können, indem die Klebestellen mit geeigneten Lösungsmitteln benetzt und beaufschlagt werden.

Beide genannten Varianten - Verklebung oder sonstige Fixierung - des unteren Teilabschnittes und des oberen Teilabschnittes sind in beliebiger Weise miteinander kombinierbar.

Bei der erfindungsgemäßen Fußgelenk-Orthese kann der obere Teilabschnitt bspw. durch ein flaches Formteil gebildet sein, wobei der obere Teilabschnitt vorzugsweise mittels einer Bandage o. dgl. Befestigung am Unterschenkel des Trägers festlegbar ist. Wie oben erwähnt, sind auch temporär wirksame und/oder wieder lösbare Verklebungen des flachen Formteils an der Haut des Unterschenkels denkbar und möglich.

Auch der untere Teilabschnitt kann durch ein flaches und an den Fuß angeformtes Formteil gebildet sein, das am Fuß und/oder an einer Fußoberbekleidung wie einem Schuh oder Stiefel fixiert oder fixierbar ist. Wie oben erwähnt, sind auch temporär wirksame und/oder wieder lösbare Verklebungen des flachen Formteils an der Haut des Fußes denkbar und möglich.

Wahlweise kann der untere Teilabschnitt nach unten hin verbreitert sein und sich auf diese Weise der Lateralseite des Fußes anpassen, insbesondere durch eine der Fußkontur folgende Wölbung.

Vorzugsweise kann der untere Teilabschnitt mit einem abgewinkelten Basisabschnitt die Fußsohle des Trägers untergreifen, was eine leichte Integration des unteren Teilabschnittes in eine Sohle und einen Schaftbereich eines Schuhs oder Stiefels ermöglicht.

Das erwähnte Gelenk kann zur beweglichen Verbindung des unteren und des oberen Teilabschnittes bspw. durch ein Kugelgelenk gebildet sein.

Wahlweise kann dieses Gelenk oder Kugelgelenk einen zusätzlichen Freiheitsgrad aufweisen, indem der Abstand der beiden Teilabschnitte senkrecht zu ihren zueinander weisenden Oberflächen innerhalb eines definierten Spielraums variabel ist. Damit kann bspw. gemeint sein, dass sich die Teile voneinander abheben können, was durch das Gelenk ermöglicht sein kann, insbesondere wenn dieses eine Längsführung aufweist, die eine Bewegungskomponente der oberen und unteren Teilabschnitte in etwa senkrecht zu ihren Flachseiten erlaubt.

Das Führungselement ermöglich darüber hinaus Bewegungen in unterschiedlichen räumlichen Richtungen. Das Führungselement ist erfindungsgemäß durch eine Führungskulisse und einem darin insbesondere leicht spielbehaftet geführten und verschiebbaren und/oder winkelig anstellbaren Zapfen gebildet. Hierbei ist es von Vorteil, wenn die Führungskulisse durch eine bogenförmige Nut gebildet ist, die den Zapfen führt.

Außerdem kann die Führungskulisse in einer vorteilhaften Ausgestaltung einen konturierten äußeren Rand aufweisen, wobei eine Randkontur den Anstellwinkel zwischen den gelenkig miteinander verbundenen Teilabschnitten definiert und begrenzt.

Darüber hinaus kann der in der Führungskulisse geführte Zapfen endseitig einen vergrößerten Kopf aufweisen, der mit der korrespondierenden Randkontur der Führungskulisse zusammenwirkt und solchermaßen für die Begrenzung der Anstellwinkel zwischen den gelenkig miteinander verbundenen Teilabschnitten sorgen kann.

Der in der Führungskulisse geführte Zapfen kann vorzugsweise einen verbreiterten Sockel oder eine Verbreiterung aufweisen, der/die mit einer Rückseite der Führungskulisse, insbesondere mit einer rückseitigen Randkontur der Führungskulisse zusammenwirkt und für die Begrenzung der Anstellwinkel zwischen den gelenkig miteinander verbundenen Teilabschnitten sorgt.

Bei der erfindungsgemäßen Fußgelenk-Orthese kann zwischen dem Gelenk und dem Führungselement bzw. dessen Führungskulisse ein definierter Mindestabstand von wenigstens 20 Millimetern liegen.

Insgesamt wird ein äußerst kompaktes und wirkungsvolles therapeutisches Instrument zur Verfügung gestellt, das sich aufgrund seiner Ausführung und Baugröße hervorragend für eine Integration in ein nur leicht modifiziertes oder minimal verändertes Schuhwerk eignet und das deshalb beim Tragen kaum störend wahrgenommen wird, so dass sich einerseits für den Nutzer ein hoher Tragekomfort ergibt. Hinzu kommt der wesentliche Vorteil für den Nutzer, dass es sich minimal eingeschränkt fühlen muss und insbesondere durch das Tragen der erfindungsgemäßen Orthese kaum das Gefühl vermittelt bekommt, einen das Gesundheitsempfinden störenden Apparat tragen zu müssen.

Sofern aus Sicht des angesprochenen Fachmannes sinnvoll miteinander kombinierbar, können einige der oder alle dieser zuvor genannten Variationen oder Ausführungsvarianten der erfindungsgemäßen Fußgelenk-Orthese wahlweise auch miteinander kombiniert werden, um das oben formulierte Ziel zumindest teilweise zu erreichen, und/oder um den gewünschten Effekt der Erfindung zu erzielen.

Die nachfolgenden Ausführungen fassen nochmal einige Aspekte der zuvor bereits in verschiedenen Ausführungsvarianten erläuterten Erfindung zusammen, konkretisieren einige Aspekte, sollen jedoch nicht im Widerspruch zu den bereits gemachten Ausführungen gesehen werden, sondern in Zusammenschau, bei Zweifeln ggf. als speziellere Ausführungsvarianten und/oder Abwandlungen. So kann, wie bereits oben mehrfach erwähnt, die erfindungsgemäße Fußgelenk-Orthese am Knöchelgelenk eines menschlichen Fußes angebracht oder fixiert werden und kann in dieser Anordnung für eine sinnvolle Begrenzung eines Bewegungsumfangs im Fußknöchelgelenk des Fußes sorgen.

Wie zudem schon erwähnt, besteht eine wichtige Aufgabe der erfindungsgemäßen Fußgelenk-Orthese zudem in einer äußeren mechanischen Stabilisierung des Bänderapparats im Fußknöchelgelenk bei gleichzeitiger Begrenzung der dem Fußgelenk möglichen Winkelstellungen, um insbesondere zu verhindern, dass zuvor stattgefundene pathologische Dehnungen bzw. Überdehnungen des Bänderapparats im betroffenen Fußgelenk oder einzelner oder mehrerer Bänder bei normalen Geh-, Schreit-, Lauf- und/oder Steigbewegungen nochmals auftreten, da solche ungewollten Bewegungen oder Winkelstellungen im Fußgelenk die allmähliche Heilung des Bänderapparates behindern können oder im ungünstigsten Fall sogar erneuten Schäden im Bänderapparat Vorschub leisten können.

Die am zu behandelnden menschlichen Fuß angeordnete oder zu befestigende Orthese umfasst mindestens zwei gelenkig miteinander verbundene Teilabschnitte, die nachfolgend nochmals im Detail erläutert werden. So umfasst die Fußgelenk-Orthese insbesondere einen unteren Teilabschnitt, der an einer Lateralseite des zu stabilisierenden Fußes anliegt und insbesondere dort fixiert ist. Außerdem umfasst die Fußgelenk-Orthese einen oberen Teilabschnitt, der an einer Lateralseite des Unterschenkels anliegt und insbesondere dort fixiert ist, und zwar in einem Bereich oberhalb eines Knöchelgelenkes des Fußes.

Zudem sind der an der Lateralseite des Unterschenkels fixierte obere Teilabschnitt und der an der Lateralseite des Fußes fixierte untere Teilabschnitt mittels eines in unmittelbarer Nähe, insbesondere unterhalb der Höhe des Knöchelgelenks befindlichen und vorzugsweise spielbehafteten Gelenks beweglich miteinander verbunden. Das Gelenk soll einerseits eine freie Beweglichkeit um eine durch das Gelenk verlaufende Achse ermöglichen. Eine solche Beweglichkeit bzw. Verschwenkbarkeit kann etwa einem Anheben des Fußes gegenüber dem Unterschenkel - auch bezeichnet als Dorsalextension - sowie einer Absenkung des Fußes gegenüber dem Unterschenkel - auch bezeichnet als Plantarflexion - entsprechen.

Darüber hinaus soll die Definition des Gelenks als spielbehaftet eine Beweglichkeit des Fußes in einer dazu ungefähr senkrechten Richtung ermöglichen, was einer Fußbewegung in Form einer Inversion (Einwärtsdrehung) oder einer Eversion (Auswärtsdrehung) im Knöchelgelenk entsprechen kann.

Das die beiden erwähnten Teile der Orthese beweglich verbindende Gelenk kann wahlweise durch ein Kugelgelenk o. dgl. gebildet sein, was die angegebenen Freiheitsgrade bei den Relativbewegungen der durch das Gelenk miteinander verbundenen unteren und oberen Teilabschnitte erlaubt. Da ein solches Kugelgelenk jedoch aufgrund seiner typischen Bauart keine Abstandsveränderungen der miteinander verbundenen unteren und oberen Teilabschnitte im Gelenk erlaubt, soll dieses Gelenk im vorliegenden Zusammenhang grundsätzlich nicht auf ein Kugelgelenk eingeschränkt sein, sondern kann auch bspw. als Bandverbindung oder durch eine andere flexible Verbindung der beiden Teile realisiert sein, denn ggf. kann es sinnvoll sein, begrenzte Abstandsveränderungen der Teile innerhalb des Gelenkes in einer definierten Richtung zuzulassen.

Darüber hinaus umfasst die Fußgelenk-Orthese vorzugsweise ein Führungselement, das proximal beabstandet zum Gelenk angeordnet ist, womit eine Positionierung oberhalb des Knöchelgelenks und näher am oberen Teilabschnitt bzw. integriert im oberen Teilabschnitt gemeint sein kann. Das Führungselement, das neben dem Gelenk eine weitere Verbindung zwischen unterem Teilabschnitt und oberem Teilabschnitt herstellt, sorgt im Wesentlichen für eine Winkel- und Wegbegrenzung der Beweglichkeit und Verschwenkbarkeit der oberen und unteren Teilabschnitte gegeneinander.

Diese Winkel- und Wegbegrenzung kann insbesondere in einer Weise realisiert sein, dass das Führungselement jeweils die maximalen Schwenkwinkel sowohl bei einer Dorsalextension als auch bei einer Plantarflexion des Fußes begrenzt, wobei diese Schwenkwinkel jeweils auf definierte Grenzwinkel eingeschränkt sind, womit eine Begrenzung der Schwenkwinkel jeweils in beide Richtungen gemeint ist.

Darüber hinaus sorgt das Führungselement zusätzlich dafür, dass sowohl ein Supinationswinkel im Knöchelgelenk bei einer Inversion des Fußes als auch ein Pronationswinkel bei einer Eversion des Fußes im Knöchelgelenk durch entsprechende Einschränkungen der Anstellwinkel zwischen oberem und unterem Teilabschnitt begrenzt werden.

Über eine konkrete Ausgestaltung der Befestigung oder Verankerung der Orthese oder ihrer gelenkig miteinander gekoppelten Einzelteile am Fuß wurde bisher noch keine Aussage getroffen. Von Vorteil kann bspw. ein flacher horizontaler Abschnitt des unteren Teilabschnittes sein, der Teil einer Sohleneinlage in einem Schuh oder einer anderen Fußoberbekleidung sein kann. Ein solcher flacher horizontaler Abschnitt, der unterhalb des Sohlenbereiches des Fußes liegen kann, kann in L-förmiger und an den Fuß angepasster Kontur in einen flachen vertikalen Bereich des unteren Teilabschnittes übergehen, der dann gelenkig mit dem oberen Teilabschnitt verbunden ist, wie sich dies weiter oben beschrieben findet.

Falls die Fußgelenk-Orthese jedoch autonom, d.h. ohne Integration in einen Schuh oder eine Fußoberbekleidung, getragen werden soll, können auch andere Befestigungsvarianten in Frage kommen, bspw. eine den Fuß umschlingende Bandage o. dgl., die mittels Klett-Verschluss dort befestigt und ggf. in ihrer Spannung angepasst werden kann. Weitere, hier nicht genannte Befestigungsvarianten sind ebenfalls denkbar und für den Fachmann aus dem gesamten Beschreibungszusammenhang ableitbar.

Der gelenkig mit dem unteren Teilabschnitt verbundene obere Teilabschnitt der Fußgelenk-Orthese befindet sich an der Lateralseite des Unterschenkels des Trägers. Auch die gelenkigen Verbindungen zwischen den Teilabschnitten (unten und oben) mittels des unteren Gelenks und des die Winkelstellungen zwischen den Teilen begrenzenden Führungselements können in unterschiedlicher Weise ausgestaltet sein, was sich für den Fachmann ebenfalls aus dem gesamten Beschreibungszusammenhang ableiten lässt.

Weiterhin kann eine Bandage vorgesehen sein, die der Verankerung des oberen Teilabschnittes am Unterschenkel des Trägers dienen kann, um die Fußgelenk-Orthese zumindest näherungsweise an ihrem vorgesehenen Ort zu positionieren und/oder zu fixieren. Die hier nicht im Detail ausgeführte Bandage kann bspw. durch einen elastischen Riemen oder ein den Zweck erfüllendes Band gebildet sein, das vorzugsweise über einen geeigneten Verschluss verfügt, um es um den Unterschenkel legen und dort weitgehend unverrutschbar fixieren zu können. Das Band bzw. die Bandage kann hierzu bspw. mit einem Klettverschluss oder einem anderen geeigneten Verschlusssystem ausgestattet sein, so dass die Orthese schnell und problemlos angelegt und wieder vom Fuß des Trägers entfernt werden kann.

Nachfolgend finden sich Ausführungen zu sinnvollen Winkelbegrenzungen bei Auf- oder Abwärtsbewegungen des Fußes. Um bei einer Überdehnung oder zu therapierenden Schädigung des Bänderapparates im Fuß, insbesondere im Bereich des Knöchelgelenks, die Dorsalextension zu begrenzen, kann die Fußgelenk-Orthese insbesondere eine Begrenzung des Schwenkwinkels auf eine Größenordnung von etwa 8 bis 25 Winkelgrade, insbesondere 10 bis 20 Winkelgrade, vorsehen. Dieser Winkel kann vorzugsweise bei einem Ausgangswert von etwa 15 Winkelgraden liegen, wobei dieser Wert bedarfsweise durch Anpassung der Orthese oder durch deren Austausch gegen eine modifizierte Orthese mit anderen Dimensionierungen des Führungselementes erhöht oder reduziert werden kann.

Da aus der orthopädischen Heilbehandlung normalerweise Werte für die Dorsalextension in der Größenordnung von etwa 15 Winkelgraden vorgegeben sind, kann eine solchermaßen eingestellte Fußgelenk-Orthese als Ausgangspunkt der Behandlung dienen und nach einiger Zeit und entsprechenden Heilungsfortschritten gegen eine Orthese mit größerem Grenzwinkel für die Dorsalextension ausgetauscht werden.

Ein Absenken des Fußes durch eine Bewegung im Knöchelgelenk kann auch als Plantarflexion bezeichnet werden. Um bei der erwähnten Überdehnung oder einem zu therapierenden Trauma des Bänderapparates im Fuß und/oder im Bereich des Knöchelgelenks die Plantarflexion zu begrenzen, kann die Fußgelenk-Orthese insbesondere eine Begrenzung des Schwenkwinkels für die Plantarflexion auf eine Größenordnung von etwa 8 bis 25 Winkelgrade, insbesondere von etwa 10 bis 20 Winkelgrade vorsehen. Dieser Winkel für die Plantarflexion kann vorzugsweise bei einem Ausgangswert von etwa 15 Winkelgraden liegen, wobei dieser Wert für die Plantarflexion bedarfsweise durch Anpassung der Orthese oder durch deren Austausch gegen eine modifizierte Orthese mit anderen Dimensionierungen des Führungselementes erhöht oder reduziert werden kann.

Da aus der orthopädischen Heilbehandlung normalerweise Winkelwerte in beide Richtungen (d.h. für die Plantarflexion als auch für die Dorsalextension) in Größenordnungen von jeweils etwa 15 Winkelgraden vorgegeben sind, kann eine solchermaßen eingestellte Fußgelenk-Orthese als Ausgangspunkt der Behandlung dienen und nach einiger Zeit und entsprechenden Heilungsfortschritten gegen eine Orthese mit größerem Grenzwinkel ausgetauscht werden.

Bei einer Plantarflexion des Fußes ergibt sich eine Absenkung und eine winkelige Anstellung des am Fuß und/oder an einem Schuh verankerten unteren Teilabschnittes, die durch die Beweglichkeit im Gelenk sowie durch entsprechende Gestaltung und Dimensionierung des Führungselementes eine Bewegungsfreiheit mit einem Grenzwinkel für die Plantarflexion zwischen 0° und etwa 15° erlaubt.

Ebenso wie die soeben erläuterten Winkelbegrenzungen durch Vorgabe von Maximalwerten für die Grenzwinkel für die Plantarflexion und für die Dorsalextension sind normalerweise vergleichbare Winkelbegrenzungen für eine Inversion sowie eine Eversion des Fußes konstruktiv vorgegeben, wobei die Einwärtsdrehung oder Inversion auch einer pathologischen Supination gleichkommen oder damit in Verbindung stehen kann, während die Auswärtsdrehung oder Eversion auch einer pathologischen Pronation des Fußes gleichkommen oder damit in Verbindung stehen kann.

Es soll an dieser Stelle vorsorglich betont sein, dass die erwähnten Komponenten der Fußgelenk-Orthese, die in der beschriebenen Weise zusammenwirken, nicht zwingend als konkrete körperliche Bauteile in der erläuterten Form zu verstehen sind, sondern dass sie als Wirkungselemente aufgefasst werden sollen, die bei der beschriebenen Positionierung der Orthese an der Lateralseite des Fußes bzw. des Unterschenkels des Trägers die hier beschriebene Wirkung entfalten kann, insbesondere durch Begrenzung der Bewegungswinkel im Knöchelgelenk bei den beschriebenen Schwenkbewegungen.

Nachfolgend werden sinnvolle Winkelbegrenzungen bei Seitwärtsbewegungen des Fußes erläutert, die durch die getragene und mit dem Fuß sowie dem Unterschenkel des Trägers befestigte Fußgelenk-Orthese realisierbar sind. So entsteht eine Einwärtsdrehung oder Inversion des Fußes durch eine Bewegung im Knöchelgelenk, die sich auch als Teil einer zu behandelnden Supination des Fußes manifestieren kann und deren Bewegungsrichtung weg von der Lateralseite und hin zu einer Medialseite des Fußes weist. Um bei einer Überdehnung oder zu therapierenden Schädigung des Bänderapparates im Fuß, insbesondere im Bereich des Knöchelgelenks, diese Inversion oder Supination zu begrenzen, kann die Fußgelenk-Orthese insbesondere eine Begrenzung des entsprechenden Schwenkwinkels auf eine Größenordnung von nicht mehr als vorzugsweise ca. 10 bis 12 Winkelgraden vorsehen, wobei dieser Supinationswinkel vorzugsweise bei einem medizinisch sinnvoll zu wählenden Ausgangswert von etwa 7 Winkelgraden liegen kann, der bedarfsweise durch Anpassung der Orthese oder durch deren Austausch gegen eine modifizierte Orthese mit anderen Dimensionierungen des Führungselementes erhöht oder reduziert werden kann.

Da aus der orthopädischen Heilbehandlung normalerweise Werte für den Supinationswinkel in der Größenordnung von möglichst nicht zu überschreitenden etwa 7 Winkelgraden vorgegeben sind, kann eine solchermaßen eingestellte Fußgelenk-Orthese als Ausgangspunkt der Behandlung dienen und nach einiger Zeit und entsprechenden Heilungsfortschritten ggf. gegen eine Orthese mit größeren Grenzwerten für den Supinationswinkel ausgetauscht werden.

Ein Auswärtsdrehen oder eine Eversion des Fußes entsteht durch eine Bewegung im Knöchelgelenk, die sich auch als Teil einer zu behandelnden Pronation des Fußes manifestieren kann und deren Bewegungsrichtung weg von der Medialseite und hin zur Lateralseite des Fußes weist. Um bei der erwähnten Überdehnung oder einem zu therapierenden Trauma des Bänderapparates im Fuß und/oder im Bereich des Knöchelgelenks die Pronation oder Eversion zu begrenzen, kann die Fußgelenk-Orthese insbesondere eine Begrenzung des Schwenkwinkels für eine Pronation auf eine Größenordnung von ca. 10 bis 20 Winkelgraden vorsehen, wobei dieser Pronationswinkel vorzugsweise bei einem Ausgangswert von nicht mehr als etwa 15 Winkelgraden liegen kann, der bedarfsweise durch Anpassung der Orthese oder durch deren Austausch gegen eine modifizierte Orthese mit anderen Dimensionierungen des Führungselementes erhöht oder reduziert werden kann.

Da aus der orthopädischen Heilbehandlung normalerweise Winkelwerte als der Ausgangsstellung in Eversionsrichtung in einer Größenordnung von etwa 15 Winkelgraden vorgegeben sind, kann eine solchermaßen eingestellte Fußgelenk-Orthese als Ausgangspunkt der Behandlung dienen und nach einiger Zeit und entsprechenden Heilungsfortschritten bedarfsweise gegen eine Orthese mit größerem Grenzwert für die Eversion ausgetauscht werden.

Nachfolgend sollen einige konkrete Ausgestaltungen der Fußgelenk-Orthese sowie ihrer zusammenwirkenden Funktionselemente erläutert werden.

So kann der untere Teilabschnitt bspw. durch ein L-förmiges flaches Bauteil gebildet sein, dessen unterer Basisabschnitt bspw. in einer Sohle eines Schuhs oder eines anderen geeigneten Fußbekleidungsstückes eingearbeitet und dort fixiert sein kann, was insbesondere einer festen räumlichen Zuordnung der Fußgelenk-Orthese zum Fuß eines Trägers oder Patienten dient. Wenn der Fuß mit seiner unteren Sohlenseite auf dem Basisabschnitt steht, so befindet sich ein ungefähr orthogonal zum horizontal angeordneten Basisabschnitt stehender Anlageabschnitt an der Lateralseite des zu stabilisierenden Fußes und liegt vorzugsweise dort an. Eine Fixierung des Anlageabschnittes am Fuß ist normalerweise nicht vorgesehen, da seine Lage durch den auf dem Basisabschnitt stehenden und diesen belastenden Fuß fixiert und räumlich definiert ist.

Der untere horizontale Basisabschnitt des unteren Teilabschnittes und der sich ungefähr im 90°-Winkel in vertikale Richtung daran anschließende Anlageabschnitt können insbesondere mit sanfter Verrundung ineinander übergehen, wobei eine solche Verrundung sinnvollerweise der typischen Kontur der Lateralseite des Fußes folgt, was sowohl den Tragekomfort der Orthese als auch deren Sitz am Fuß begünstigt.

Der in etwa vertikal verlaufende Anlageabschnitt kann eine dem Fuß im Höhe des Knöchelbereiches zugewandte konkave Einwölbung aufweisen, um der Fußkontur besser zu folgen und um insbesondere nach Möglichkeit Druckstellen und/oder eine schlechte Passform der Orthese zu vermeiden, denn der gesamte untere Teilabschnitt liegt weitgehend flächig am Fuß des Trägers an und verläuft hierbei von der Sohle bis knapp oberhalb des Knöchelbereiches, wo der in etwa vertikal stehende Anlageabschnitt endet.

Weiterhin kann der untere Teilabschnitt als flacher Streifen ausgebildet sein, der eine deutlich größere Breite als Dicke aufweist. Die Dicke oder Materialstärke des Streifens kann so gering wie möglich ausfallen, um den Tragekomfort und die Integrierbarkeit in einen Schuh oder Stiefel zu verbessern, muss jedoch ausreichend stabil sein, um zu verhindern, dass sich der untere Teilabschnitt beim Tragen unter den Fußbewegungen zu stark verformen kann. Sollte dies der Fall sein, so könnten die angestrebten mechanischen Funktionen, die im Wesentlichen in der Begrenzung der Bewegungswinkel zwischen den gelenkig miteinander verbundenen Teilabschnitten liegen, beeinträchtigt sein.

Die Breite eines solchen Streifens, der den unteren Teilabschnitt bildet, richtet sich in erster Linie nach der gewünschten Aufstandsfläche des horizontalen Basisabschnittes, an der gewünschten Anlagefläche des sich in vertikale Richtung über die Verrundung an den Basisabschnitt anschließenden Anlageabschnittes sowie an den im unteren Teilabschnitt unterzubringenden Komponenten und Wirkelementen des Gelenks sowie des Führungselementes.

Eine sinnvolle Breite des unteren Teilabschnittes kann bspw. bei ca. zwei bis fünf Zentimetern liegen, während eine sinnvolle Materialstärke vom verwendeten Material abhängt. Ein hochfestes Fasermaterial wie bspw. CFK (mit Kohlefasern verstärkter Kunststoff) oder auch GFK (mit Glasfasern verstärkter Kunststoff) ermöglicht einen relativ dünn ausgeführten Streifen mit einer Materialstärke von deutlich unter zwei Millimetern. Gleiches gilt für ein hochfestes und ausreichend flexibles Metallmaterial wie bspw. eine geeignete Aluminium- oder Titanlegierung, die sehr dünne Materialstärken von weniger als einem Millimeter erlaubt.

An der vom Fuß abgewandten Außenseite des Anlageabschnittes, etwa in Höhe des Knöchelbereiches des Fußes und damit an der gegenüberliegenden Seite der optional zu verstehenden konkaven Einwölbung, befindet sich der dem unteren Teilabschnitt zugeordnete Bereich des Gelenks, der die beiden Teilabschnitte gelenkig miteinander verbindet.

Wie oben erwähnt, könnte das Gelenk wahlweise als Kugelgelenk ausgebildet sein, so dass der untere Teilabschnitt z.B. mit einer Gelenkpfanne auszustatten wäre, in der sich eine mit dem oberen Teilabschnitt verbundene Gelenkkugel weitgehend spielfrei drehen könnte, so dass eine allseitige Verdrehung um den Drehpunkt eines solchen Kugelgelenks ermöglicht wäre.

Stattdessen kann sich auf der konvex ausgewölbten Seite des Anlageabschnittes ein ungefähr senkrecht nach außen weisender Stift befinden, der an der konvexen Auswölbung des Anlageabschnittes verankert sein kann und somit in etwa horizontal verläuft. Der Stift kann mittig oder auch außermittig aus einem Sockel herausragen, der vorzugsweise die konvexe Auswölbung fortsetzt und dem Stift solchermaßen eine stabile Verankerung bietet, so dass er gewissen mechanischen Belastungen standhalten kann, die bei der Verwendung der Fußgelenk-Orthese auftreten können.

Der Stift führt den vorzugsweise mit passender Bohrung ausgestatteten oberen Teilabschnitt, wobei die Bohrung sinnvollerweise einen größeren Bohrungsdurchmesser aufweisen sollte als der Außendurchmesser des Stiftes, so dass ein ausreichendes Spiel für Verschwenkungen und Verkippungen des am Stift als Gelenkachse aufgehängten und geführten oberen Teilabschnittes der Orthese gegeben ist. Außerdem erlaubt es die lose Längsführung der Bohrung auf dem Stift, dass der Abstand des oberen Teilabschnittes im Bereich dieser gelenkigen Lagerung (Gelenk) zum unteren Teilabschnitt in gewissen Grenzen variabel bleibt.

Der maximale Abstand der beiden Abschnitte zueinander ist jedoch durch die nachfolgend beschriebene Ausgestaltung und Funktionsweise des mit dem Gelenk zusammenwirkenden und vom Gelenk beabstandeten Führungselementes vorgegeben, während sich der minimale Abstand beim vollständigen Aufschieben des oberen Teilabschnittes mit seiner am Stift geführten Bohrung auf den Sockel einstellt.

Die Fußgelenk-Orthese umfasst neben dem ausführlich in seinen einzelnen Elementen erläuterten unteren Teilabschnitt den über das Gelenk und das Führungselement damit verbundenen oberen Teilabschnitt, der an der Lateralseite des Unterschenkels des Trägers anliegt und insbesondere dort fixiert ist, und zwar in einem Bereich deutlich oberhalb des Knöchelgelenkes des Fußes.

Das Führungselement ist proximal beabstandet zum Gelenk angeordnet, womit eine Positionierung oberhalb des Knöchelgelenks und näher am oberen Teilabschnitt bzw. integriert im oberen Teilabschnitt gemeint ist. Das Führungselement, das neben dem Gelenk eine weitere Verbindung zwischen unterem Teilabschnitt und oberem Teilabschnitt herstellt, sorgt im Wesentlichen für die gewünschte Winkel- und Wegbegrenzung der Beweglichkeit und Verschwenkbarkeit der oberen und unteren Teilabschnitte gegeneinander.

Das Führungselement, dessen Wirkungsweise hinsichtlich der zu begrenzenden Schwenkwinkel bei einer Plantarflexion und einer Dorsalextension bereits in seinen Grundsätzen weiter oben erläutert wurde, wird nachfolgend anhand einer konkreten Ausführungsvariante bzw. konstruktiven und fertigungsmäßigen Ausgestaltung beschrieben. So kann etwa das Führungselement durch einen starr am oberen Bereich des Anlageabschnittes verankerten zylindrischen Zapfen mit pilzförmiger Verbreiterung am freien Ende, der in einer nutartigen Führungskulisse des oberen Teilabschnittes geführt ist und dort entlanggleiten kann, gebildet sein. Hierbei und hierdurch kann die zum Zapfen weisende Stirnseite der pilzförmigen Verbreiterung auf einem konturierten und über eine definierte Schrägrampe o. dgl. verfügenden erhöhten äußeren Rand der Führungskulisse geführt und damit in seiner Bewegungsfreiheit eingeschränkt sein.

Außerdem kann eine beabstandet zur endseitigen pilzförmigen Verbreiterung befindliche weitere scheibenförmige Verbreiterung vorgesehen sein, die den Zapfen seiner Länge nach in etwa mittig unterteilt, wodurch sich ausschließlich ein zwischen den beiden Verbreiterungen befindlicher äußerer hülsenförmiger oder zylindrischer Abschnitt des Zapfens innerhalb der Führungskulisse bewegen kann, während ein zwischen dem Anlageabschnitt und der weiteren scheibenförmigen Verbreiterung liegender innerer Abschnitt des Zapfens vorzugsweise nicht mit dem oberen Teilabschnitt oder der darin befindlichen Führungskulisse in Kontakt kommt.

Der innere Abschnitt dient typischerweise lediglich der festen Verankerung des Zapfens im oberhalb des Sockels mit dem Stift befindlichen oberen Bereich des Anlageabschnittes des unteren Teilabschnittes, während die Länge des Zapfens und die Positionen der daran angeordneten Verbreiterungen im Zusammenhang mit der Konturierung des die Schrägrampe bildenden umgebenden Randes der Führungskulisse die Winkelbegrenzungen bei einer Pronation oder Supination des Fußes definieren.

So definiert die insbesondere den gesamten umgebenden Rand der Führungskulisse bildende Schrägrampe, die die äußere pilzförmige Verbreiterung am Zapfen führt, im Wesentlichen die maximalen Supinationswinkel, die durch die Kontur, den Verlauf und die Höhe der Schrägrampe eingeschränkt sind. Mögliche und sinnvolle Gestaltungen der Schrägrampe werden weiter unten näher erläutert.

Dagegen werden die maximalen Pronationswinkel durch eine Kontur einer die Führungskulisse zur Innenseite umgebenden Randgestaltung definiert, die eine Führung für die weitere scheibenförmige Verbreiterung des Zapfens bildet, wobei diese innere Randgestaltung der Führungskulisse ebenfalls weiter unten näher erläutert wird.

Das Zusammenspiel des bei am Fuß getragener Orthese ungefähr in Höhe des Knöchelgelenks befindlichen Gelenks mit dem in proximaler Richtung beabstandet dazu angeordneten Führungselement sorgt für die gewünschten Winkelbegrenzungen sowohl bei einer Plantarflexion als auch bei einer Dorsalextension. Die maximalen Schwenkwinkel für die Dorsalextension sowie für die Plantarflexion sind jeweils durch die Dimensionierung der mit dem Zapfen zusammenwirkenden Führungskulisse definiert und begrenzt.

Bei annähernd fluchtenden unteren und oberen Abschnitten ist der Fuß weder nennenswert nach oben gestreckt noch nennenswert nach unten gebeugt. Der zylindrische Zapfen kann sich hierbei ungefähr mittig in der länglichen und einem leicht gekrümmten Verlauf folgenden Führungskulisse befinden, wobei die stirnseitige pilzartige Verbreiterung des Zapfens insbesondere auf dem weiter unten erläuterten erhöhten äußeren Rand mit seiner Schrägrampe aufliegen kann. Da jedoch der zwischen den beiden Verbreiterungen befindliche äußere zylindrische Abschnitt des Zapfens mit geringem Spiel in der Führungskulisse gleiten kann, gibt diese die Beweglichkeit der Teilabschnitte zueinander vor.

Bei einer ersten Schwenkposition des oberen Teilabschnittes gegen den unteren Teilabschnitt um das untere Gelenk ist der Fuß angehoben. Da sich die Bohrung des oberen Teilabschnittes auf dem Stift des unteren Teilabschnittes befindet, bildet diese Passung ein Drehgelenk mit einer Drehachse, die mittig durch den Stift bzw. die Bohrung verläuft, so dass die Verschwenkung der beiden Teilabschnitte zueinander in der gezeigten Weise erfolgen kann. Die Krümmung der länglichen Führungskulisse hat demzufolge ihren Mittelpunkt in etwa in dieser durch den Stift bzw. durch die Bohrung definierten Drehachse.

Der erhöhte äußere Rand der Führungskulisse kann konturiert sein. Eine solche Konturierung kann hinsichtlich der Anlageführung für die pilzförmige äußere Verbreiterung, die den Zapfen endseitig abschließt, für die Winkelbegrenzungen bei einer Inversion oder Supination des Fußes sorgen. Bei einem stärker erhöhten Rand und entsprechend steilerer Schrägrampe verbleibt ein geringerer axialer Bewegungsspielraum für den zwischen den Verbreiterungen befindlichen äußeren Zapfenabschnitt, wodurch auch der Supinationswinkel stärker begrenzt wird als bei einem flacher gestalteten Rand der Führungskulisse.

Außerdem kann auch der als zusätzliche Führungsnut gestaltete innere Rand der Führungskulisse konturiert sein. Eine solche Konturierung kann eine Breite aufweisen, welche dem Außendurchmesser der weiteren scheibenförmigen Verbreiterung des Zapfens entspricht, so dass diese Verbreiterung parallel zur Führung des Zapfens innerhalb der Öffnung der Führungskulisse beitragen kann. Die Tiefe der zusätzlichen Führungsnut und damit die verbleibende Stärke der Führungskulisse definiert/definieren wiederum den axialen Bewegungsspielraum für den zwischen den Verbreiterungen befindlichen äußeren Zapfenabschnitt, wobei der Pronationswinkel durch die Anlage der Verbreiterung im Grund der Führungsnut begrenzt wird, denn bei einer weniger starken Vertiefung der Führungsnut verbleibt ein geringerer axialer Bewegungsspielraum für den zwischen den Verbreiterungen befindlichen äußeren Zapfenabschnitt, wodurch auch der Pronationswinkel stärker begrenzt wird als bei einer tiefer ausgeführten Führungsnut an der Rückseite der Führungskulisse.

Die Länge der Führungskulisse, die Höhe des äußeren Randes sowie die Tiefe der die Führungskulisse an der Innenseite umgebenden zusätzlichen Führungsnut definieren den Bewegungsspielraum bei den erläuterten Fußbewegungen, wenn die Fußgelenk-Orthese getragen wird.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

Figuren 1A bis 1C zeigen in schematischen Seitenansichten die Bewegungsspielräume einer Ausführungsvariante einer Fußgelenk-Orthese bei im Knöchelgelenk angehobenem oder abgesenktem Fuß.

Figuren 2A bis 2C zeigen in schematischen Vorderansichten die Bewegungsspielräume der Fußgelenk-Orthese bei im Knöchelgelenk eingedrehtem oder ausgedrehtem Fuß.

Figuren 3A bis 3C zeigen eine Ausführungsvariante einer erfindungsgemäßen Fußgelenk-Orthese in verschiedenen perspektivischen Ansichten.

Figuren 4A bis 4D zeigen die Fußgelenk-Orthese in verschiedenen Winkelstellungen.

Figuren 5A und 5B zeigen eine Führungskulisse der Fußgelenk-Orthese, durch deren Gestaltung die maximalen Schwenkwinkel der verschiedenen Winkelstellungen definiert werden.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die Erfindung ausgestaltet sein kann und stellen keine abschließende Begrenzung dar. Auch sind die nachfolgend beschriebenen Merkmale jeweils nicht in engem Zusammenhang mit weiteren Merkmalen des jeweiligen Ausführungsbeispiels zu verstehen, sondern können jeweils im allgemeinen Zusammenhang vorgesehen sein bzw. hierfür Verwendung finden.

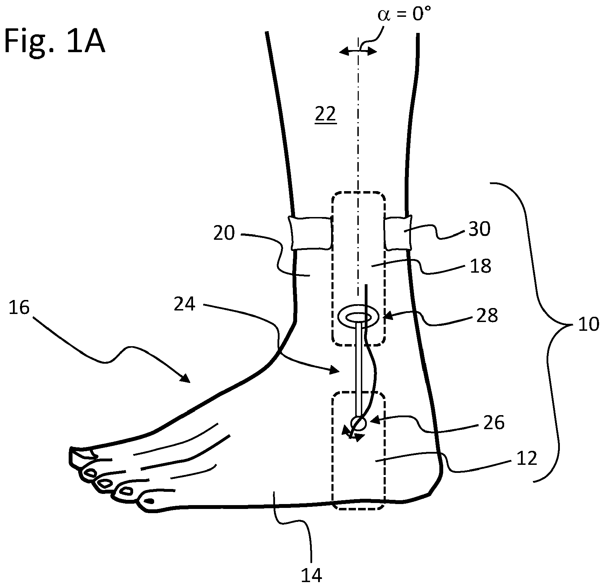

Die schematische Seitenansicht der Fig. 1A zeigt anhand einer ersten Ausführungsvariante die wesentlichen Funktionen einer erfindungsgemäßen Fußgelenk-Orthese, die am Knöchelgelenk eines menschlichen Fußes angebracht oder fixiert werden kann und in dieser Anordnung für eine Begrenzung eines Bewegungsumfangs im Fußknöchelgelenk des Fußes sorgen kann. Eine wichtige Aufgabe der erfindungsgemäßen Fußgelenk-Orthese besteht zudem in einer äußeren mechanischen Stabilisierung des Bänderapparats im Fußknöchelgelenk bei gleichzeitiger Begrenzung der dem Fußgelenk möglichen Winkelstellungen, um insbesondere zu verhindern, dass zuvor stattgefundene pathologische Dehnungen bzw. Überdehnungen des Bänderapparats im betroffenen Fußgelenk oder einzelner oder mehrerer Bänder nochmals auftreten, da solche ungewollten Bewegungen oder Winkelstellungen im Fußgelenk die allmähliche Heilung des Bänderapparates behindern können oder im ungünstigsten Fall sogar erneuten Schäden im Bänderapparat Vorschub leisten können.

Die hier am menschlichen Fuß angeordnete oder zu befestigende Orthese umfasst mindestens zwei gelenkig miteinander verbundene Teilabschnitte, die nachfolgend näher erläutert und anhand der folgenden Figuren in weiteren Details gezeigt werden. So umfasst die hier allgemein mit der Bezugsziffer 10 gekennzeichnete Fußgelenk-Orthese einen unteren Teilabschnitt 12, der an einer Lateralseite 14 des zu stabilisierenden Fußes 16 anliegt und insbesondere dort fixiert ist.

Außerdem umfasst die Fußgelenk-Orthese 10 einen oberen Teilabschnitt 18, der an einer Lateralseite 20 des Unterschenkels 22 anliegt und insbesondere dort fixiert ist, und zwar in einem Bereich oberhalb eines Knöchelgelenkes 24 des Fußes 16.

Wie es die Fig. 1A weiterhin in schematischer Weise verdeutlicht, sind der an der Lateralseite 20 des Unterschenkels 22 fixierte obere Teilabschnitt 18 und der an der Lateralseite 14 des Fußes 16 fixierte untere Teilabschnitt 12 mittels eines in unmittelbarer Nähe, insbesondere unterhalb der Höhe des Knöchelgelenks 24 befindlichen und vorzugsweise spielbehafteten Gelenks 26 beweglich miteinander verbunden. Das Gelenk 26 soll einerseits eine freie Beweglichkeit um eine durch das Gelenk 26 verlaufende und insbesondere aus der Zeichnungsebene herausragenden Achse (nicht dargestellt) ermöglichen, was durch den Doppelpfeil am Gelenk 26 angedeutet ist. Eine solche Beweglichkeit bzw. Verschwenkbarkeit kann etwa einem Anheben des Fußes 16 gegenüber dem Unterschenkel 22 - auch bezeichnet als Dorsalextension - sowie einer Absenkung des Fußes 16 gegenüber dem Unterschenkel 22 - auch bezeichnet als Plantarflexion - entsprechen.

Darüber hinaus soll die Definition des Gelenks 26 als spielbehaftet eine Beweglichkeit des Fußes 16 in einer dazu ungefähr senkrechten Richtung, d.h. um eine innerhalb der Zeichnungsebene liegenden Schwenkachse (ebenfalls nicht dargestellt) ermöglichen, was einer Fußbewegung in Form einer Inversion (Einwärtsdrehung) oder einer Eversion (Auswärtsdrehung) im Knöchelgelenk 24 entsprechen kann.

Das die beiden Teile 12 und 18 der Orthese 10 beweglich verbindende Gelenk 26 kann wahlweise durch ein Kugelgelenk o. dgl. gebildet sein, was die angegebenen Freiheitsgrade bei den Relativbewegungen der durch das Gelenk 26 miteinander verbundenen unteren und oberen Teilabschnitte 12 und 18 erlaubt. Da ein solches Kugelgelenk jedoch aufgrund seiner typischen Bauart keine Abstandsveränderungen der miteinander verbundenen unteren und oberen Teilabschnitte 12 und 18 im Gelenk 26 erlaubt, soll dieses Gelenk 26 grundsätzlich nicht auf ein Kugelgelenk eingeschränkt sein, sondern kann auch bspw. als Bandverbindung oder durch eine andere flexible Verbindung der beiden Teile 12 und 18 realisiert sein, denn ggf. kann es sinnvoll sein, begrenzte Abstandsveränderungen der Teile 12 und 18 innerhalb des Gelenkes 26 in einer aus der Zeichnungsebene weisenden Richtung zuzulassen.

Darüber hinaus umfasst die hier gezeigte Fußgelenk-Orthese 10 ein Führungselement 28, das proximal beabstandet zum Gelenk 26 angeordnet ist, womit eine Positionierung oberhalb des Knöchelgelenks 24 und näher am oberen Teilabschnitt 18 bzw. integriert im oberen Teilabschnitt 18 gemeint sein kann. Das Führungselement 28, das neben dem Gelenk 26 eine weitere Verbindung zwischen unterem Teilabschnitt 12 und oberem Teilabschnitt 18 herstellt, sorgt im Wesentlichen für eine Winkel- und Wegbegrenzung der Beweglichkeit und Verschwenkbarkeit der oberen und unteren Teilabschnitte 12 und 18 gegeneinander.

Diese Winkel- und Wegbegrenzung ist in einer Weise realisiert, dass das Führungselement 28 jeweils die maximalen Schwenkwinkel sowohl bei einer Dorsalextension als auch bei einer Plantarflexion des Fußes 16 begrenzt, wobei diese Schwenkwinkel jeweils auf definierte Grenzwinkel eingeschränkt sind, womit eine Begrenzung der Schwenkwinkel jeweils in beide Richtungen gemeint ist.

Darüber hinaus sorgt das Führungselement 28 zusätzlich dafür, dass sowohl ein Supinationswinkel im Knöchelgelenk 24 bei einer Inversion des Fußes 16 als auch ein Pronationswinkel bei einer Eversion des Fußes 16 im Knöchelgelenk 24 durch entsprechende Einschränkungen der Anstellwinkel zwischen oberem und unterem Teilabschnitt 18 und 12 begrenzt werden.

Die Seitenansicht der Fig. 1A zeigt die Anordnung der Fußgelenk-Orthese 10 am linken Fuß 16 eines Menschen, wobei sich der untere Teilabschnitt 12 der Orthese 10 an der Lateralseite 14 des Fußes 16 befindet, ohne dass hier eine nähere Ausgestaltung der Befestigung oder Verankerung am Fuß 16 dargestellt ist. Von Vorteil kann bspw. ein flacher horizontaler Abschnitt des unteren Teilabschnittes 12 sein, der Teil einer Sohleneinlage in einem Schuh (nicht gezeigt) oder einer anderen Fußoberbekleidung sein kann. Ein solcher flacher horizontaler Abschnitt, der unterhalb des Sohlenbereiches des Fußes 16 liegen kann, kann in L-förmiger und an den Fuß 16 angepasster Kontur in den in Fig. 1A schematisch gezeigten flachen vertikalen Bereich des unteren Teilabschnittes 12 übergehen, der dann gelenkig mit dem oberen Teilabschnitt 18 verbunden ist, wie sich dies weiter oben beschrieben findet.

Falls die Fußgelenk-Orthese 10 jedoch autonom, d.h. ohne Integration in einen Schuh oder eine Fußoberbekleidung, getragen werden soll, können auch andere Befestigungsvarianten in Frage kommen, bspw. eine den Fuß 16 umschlingende Bandage o. dgl., die mittels Klett-Verschluss dort befestigt und ggf. in ihrer Spannung angepasst werden kann. Weitere, hier nicht genannte Befestigungsvarianten sind ebenfalls denkbar.

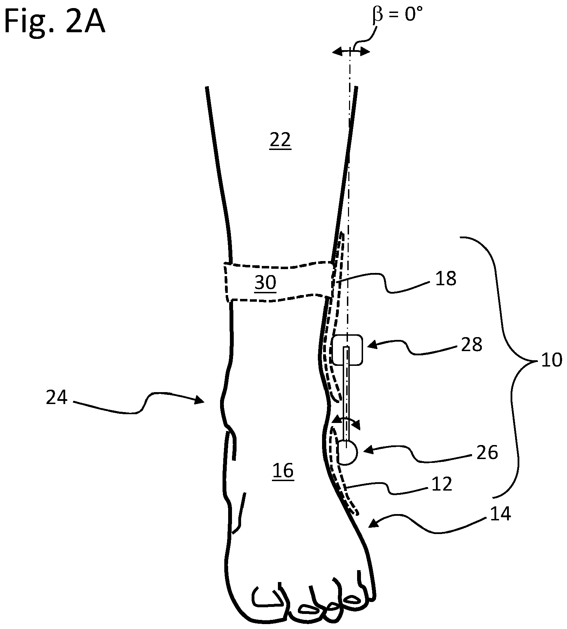

Der gelenkig mit dem unteren Teilabschnitt 12 verbundene obere Teilabschnitt 18 der Fußgelenk-Orthese 10 befindet sich an der Lateralseite 20 des Unterschenkels 22 des Trägers, was in der Seitenansicht der Fig. 1A und in der schematischen Frontansicht auf den linken Fuß 16 der Fig. 2A verdeutlicht ist. Die gelenkigen Verbindungen zwischen den Teilabschnitten 12 (unten) und 18 (oben) mittels des unteren Gelenks 26 und des die Winkelstellungen zwischen den Teilen 12 und 18 begrenzenden Führungselements 28 sind in den Figuren 1A und 2A lediglich schematisch angedeutet und nicht in ihrer körperlichen Ausgestaltung dargestellt.

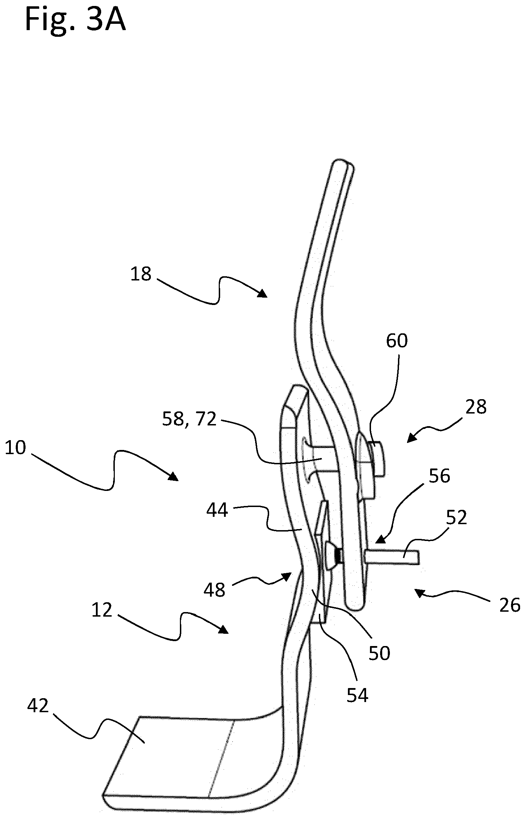

Eine konkretere, aber ebenfalls beispielhaft anzusehende Ausgestaltung der Fußgelenk-Orthese 10 sowie ihrer zusammenwirkenden Funktionselemente findet sich in den Figuren 3A bis 5B, die weiter unten näher erläutert werden.

Weiterhin verdeutlichen die Seitenansicht der Fig. 1A sowie die Frontansicht der Fig. 2A in schematischer Weise eine Bandage 30, die der Verankerung des oberen Teilabschnittes 18 am Unterschenkel 22 des Trägers dienen kann, um die Fußgelenk-Orthese 10 zumindest näherungsweise an ihrem vorgesehenen Ort zu positionieren und zu fixieren. Die hier nicht im Detail ausgeführte Bandage 30 kann bspw. durch einen elastischen Riemen oder ein den Zweck erfüllendes Band gebildet sein, das über einen geeigneten Verschluss verfügt, um es um den Unterschenkel 22 legen und dort weitgehend unverrutschbar fixieren zu können. Das Band bzw. die Bandage 30 kann hierzu bspw. mit einem Klettverschluss oder einem anderen geeigneten Verschlusssystem ausgestattet sein, so dass die Orthese 10 schnell und problemlos angelegt und wieder vom Fuß 16 des Trägers entfernt werden kann.

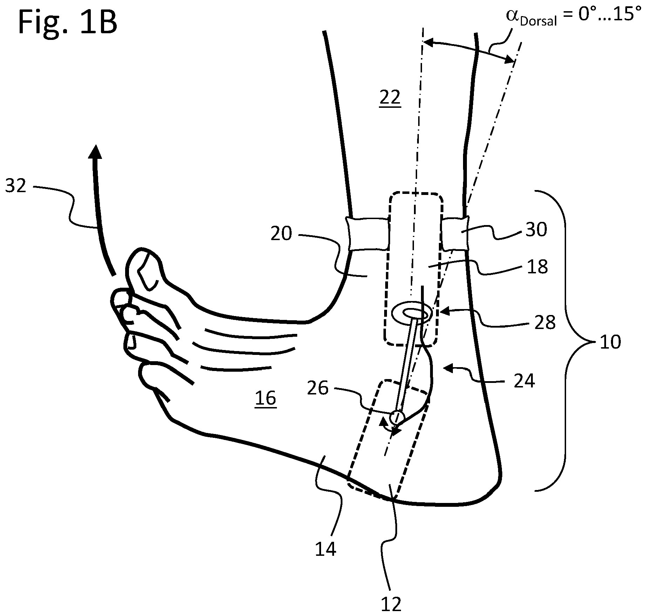

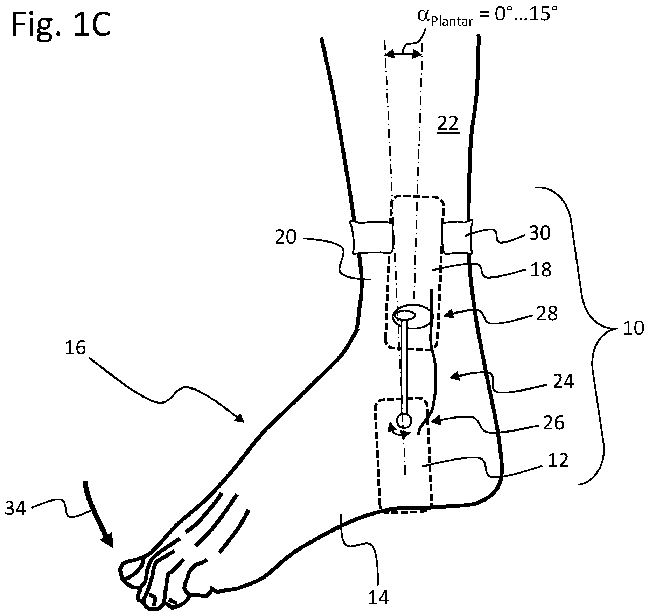

Anhand der weiteren schematischen Seitenansichten der Figuren 1 B und 1C sollen sinnvolle Winkelbegrenzungen bei Auf- oder Abwärtsbewegungen des Fußes 16 veranschaulicht werden, die durch die getragene und mit dem Fuß 16 sowie dem Unterschenkel 22 des Trägers befestigte Fußgelenk-Orthese 10 realisierbar sind.

So verdeutlicht die Fig. 1B ein Anheben des Fußes 16 durch eine Bewegung im Knöchelgelenk 24, die auch als Dorsalextension bezeichnet und deren Bewegungsrichtung durch einen mit der Bezugsziffer 32 gekennzeichneten Pfeil nach oben veranschaulicht wird. Um bei einer Überdehnung oder zu therapierenden Schädigung des Bänderapparates im Fuß 16, insbesondere im Bereich des Knöchelgelenks 24, die Dorsalextension 32 zu begrenzen, kann die gezeigte Fußgelenk-Orthese 10 insbesondere eine Begrenzung des Schwenkwinkels α_{Dorsal} auf eine Größenordnung von ca. 10° bis 20° vorsehen, wobei dieser Winkel α_{Dorsal} vorzugsweise bei einem Ausgangswert von etwa 15° liegen kann, der bedarfsweise durch Anpassung der Orthese 10 oder durch deren Austausch gegen eine modifizierte Orthese 10 mit anderen Dimensionierungen des Führungselementes 28 erhöht oder reduziert werden kann.

Da aus der orthopädischen Heilbehandlung normalerweise Werte in der Größenordnung von α_{Dorsal} ≈ 15° vorgegeben sind, kann eine solchermaßen eingestellte Fußgelenk-Orthese 10 als Ausgangspunkt der Behandlung dienen und nach einiger Zeit und entsprechenden Heilungsfortschritten gegen eine Orthese 10 mit größerem Grenzwinkel für α_{Dorsal} ausgetauscht werden.

Wie schon oben mehrfach beschrieben, zeigt die Fig. 1A zeigt einen gerade stehenden Fuß 16 von der Seite, der weder nach oben angehoben noch nach unten abgesenkt ist. Der Schwenkwinkel α, der zwischen dem unteren Teilabschnitt 12 und dem oberen Teilabschnitt 18 auftritt, weist dort demnach einen Wert von Null auf (α = 0°; vgl. Fig. 1A). Dagegen verdeutlicht die Fig. 1 B eine Dorsalextension 32 des Fußes 16, woraus sich eine winkelige Anstellung des am Fuß 16 und/oder an einem Schuh (nicht gezeigt) verankerten unteren Teilabschnittes 12 ergibt, die durch Beweglichkeit im Gelenk 26 sowie durch entsprechende Gestaltung und Dimensionierung des Führungselementes 28 in der zeigten Weise eine Bewegungsfreiheit mit einem Grenzwinkel α_{Dorsal} für die Dorsalextension 32 zwischen 0° und etwa 15° (α_{Dorsal} = 0° ... 15°) erlaubt.

Weiterhin verdeutlicht die Fig. 1C ein Absenken des Fußes 16 durch eine Bewegung im Knöchelgelenk 24, die auch als Plantarflexion bezeichnet und deren Bewegungsrichtung durch einen mit der Bezugsziffer 34 gekennzeichneten Pfeil nach unten veranschaulicht wird. Um bei der erwähnten Überdehnung oder einem zu therapierenden Trauma des Bänderapparates im Fuß 16 und/oder im Bereich des Knöchelgelenks 24 die Plantarflexion 34 zu begrenzen, kann die gezeigte Fußgelenk-Orthese 10 insbesondere eine Begrenzung des Schwenkwinkels αₚₗₐₙₜₐᵣ auf eine Größenordnung von ca. 10° bis 20° vorsehen, wobei dieser Winkel αₚₗₐₙₜₐᵣ vorzugsweise bei einem Ausgangswert von etwa 15° liegen kann, der bedarfsweise durch Anpassung der Orthese 10 oder durch deren Austausch gegen eine modifizierte Orthese 10 mit anderen Dimensionierungen des Führungselementes 28 erhöht oder reduziert werden kann.

Da aus der orthopädischen Heilbehandlung normalerweise Winkelwerte in beide Richtungen in Größenordnungen von jeweils etwa 15° (d.h. α_{Dorsal} ≈ 15° und α_{Plantar} ≈ 15°) vorgegeben sind, kann eine solchermaßen eingestellte Fußgelenk-Orthese 10 als Ausgangspunkt der Behandlung dienen und nach einiger Zeit und entsprechenden Heilungsfortschritten gegen eine Orthese 10 mit größerem Grenzwinkel für αₚₗₐₙₜₐᵣ ausgetauscht werden.

Wie erwähnt, verdeutlicht die Fig. 1C eine Plantarflexion 34 des Fußes 16, woraus sich eine Absenkung und eine winkelige Anstellung des am Fuß 16 und/oder an einem Schuh (nicht gezeigt) verankerten unteren Teilabschnittes 12 ergibt, die durch die Beweglichkeit im Gelenk 26 sowie durch entsprechende Gestaltung und Dimensionierung des Führungselementes 28 in der zeigten Weise eine Bewegungsfreiheit mit einem Grenzwinkel α_{Plantar} für die Plantarflexion 34 zwischen 0° und etwa 15° (α_{Plantar} = 0° ... 15°) erlaubt.

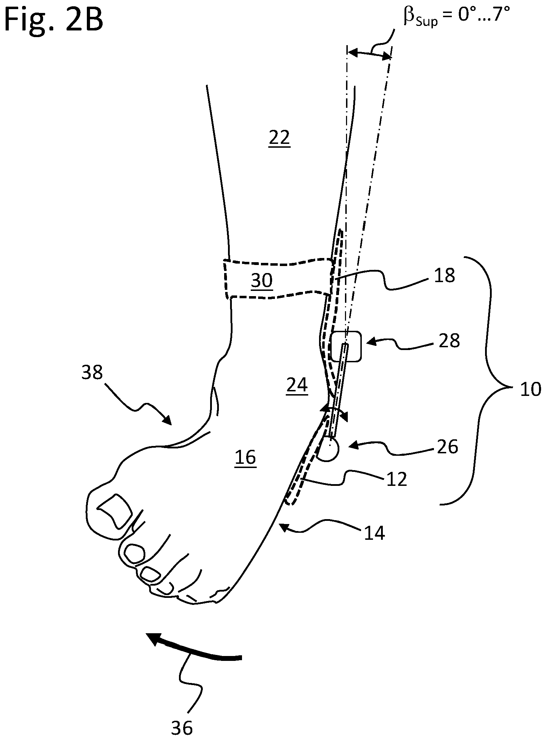

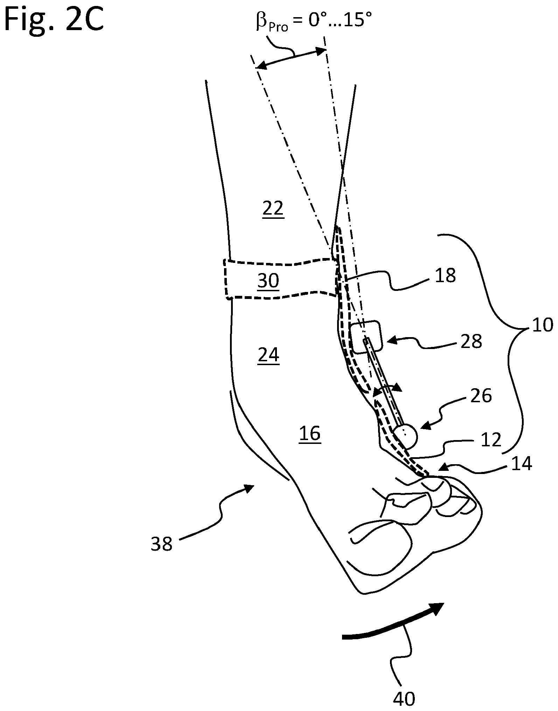

Ebenso wie die anhand der Figuren 1A bis 1C verdeutlichten Winkelbegrenzungen durch Vorgabe von Maximalwerten für die Grenzwinkel α_{Plantar} für die Plantarflexion 34 (vgl. Fig. 1C) und α_{Dorsal} für die Dorsalextension 32 sollen die Figuren 2B und 2C vergleichbare Winkelbegrenzungen für eine Inversion sowie eine Eversion des Fußes 16, wobei die Einwärtsdrehung oder Inversion auch einer pathologischen Supination gleichkommen oder damit in Verbindung stehen kann, und wobei die Auswärtsdrehung oder Eversion auch einer pathologischen Pronation des Fußes gleichkommen oder damit in Verbindung stehen kann.

Es soll an dieser Stelle vorsorglich nochmals dasselbe betont sein, wie es schon weiter oben in Bezug auf die Figuren 1A bis 1C erwähnt wurde, dass die in den Figuren 2A bis 2C schematisch gezeigten Komponenten der Fußgelenk-Orthese 10, die in der beschriebenen Weise zusammenwirken, nicht zwingend als konkrete körperliche Bauteile in der zeichnerisch dargestellten Form zu verstehen sind, sondern dass sie als Wirkungselemente aufgefasst werden sollen, die bei der beschriebenen Positionierung der Orthese 10 an der Lateralseite 14 bzw. 20 des Fußes 16 bzw. des Unterschenkels 22 des Trägers die hier beschriebene Wirkung entfalten kann, insbesondere durch Begrenzung der Bewegungswinkel im Knöchelgelenk 24 bei den beschriebenen Schwenkbewegungen.

Anhand der weiteren schematischen Seitenansichten der Figuren 2B und 2C sollen sinnvolle Winkelbegrenzungen bei Seitwärtsbewegungen des Fußes 16 veranschaulicht werden, die durch die getragene und mit dem Fuß 16 sowie dem Unterschenkel 22 des Trägers befestigte Fußgelenk-Orthese 10 realisierbar sind.

So verdeutlicht die Fig. 2B eine Einwärtsdrehung oder Inversion des Fußes 16 durch eine Bewegung im Knöchelgelenk 24, die sich auch als Teil einer zu behandelnden Supination des Fußes 16 manifestieren kann und deren Bewegungsrichtung durch einen mit der Bezugsziffer 36 gekennzeichneten Pfeil weg von der Lateralseite 14 und hin zu einer Medialseite 38 des Fußes 16 veranschaulicht wird. Um bei einer Überdehnung oder zu therapierenden Schädigung des Bänderapparates im Fuß 16, insbesondere im Bereich des Knöchelgelenks 24, diese Inversion oder Supination 36 zu begrenzen, kann die gezeigte Fußgelenk-Orthese 10 insbesondere eine Begrenzung des in Fig. 2B eingezeichneten Schwenkwinkels β_{Sup} auf eine Größenordnung von nicht mehr als ca. 10° bis 12° vorsehen, wobei dieser Supinationswinkel β_{Sup} vorzugsweise bei einem medizinisch sinnvoll zu wählenden Ausgangswert von etwa 7° liegen kann, der bedarfsweise durch Anpassung der Orthese 10 oder durch deren Austausch gegen eine modifizierte Orthese 10 mit anderen Dimensionierungen des Führungselementes 28 erhöht oder reduziert werden kann.

Da aus der orthopädischen Heilbehandlung normalerweise Werte für den Supinationswinkel in der Größenordnung von möglichst nicht zu überschreitenden etwa 7° (ß_{Sup} ≈ 7°) vorgegeben sind, kann eine solchermaßen eingestellte Fußgelenk-Orthese 10 als Ausgangspunkt der Behandlung dienen und nach einiger Zeit und entsprechenden Heilungsfortschritten ggf. gegen eine Orthese 10 mit größerem Grenzwinkel für β_{Sup} ausgetauscht werden.

Wie schon oben mehrfach beschrieben, zeigt die Fig. 2A zeigt einen auf einer ebenen Unterlage gerade stehenden Fuß 16 von vorne, der weder nach oben angehoben, nach unten abgesenkt, nach innen eingebogen oder nach außen gedreht ist. Der Schwenkwinkel β, der zwischen dem unteren Teilabschnitt 12 und dem oberen Teilabschnitt 18 der Orthese 10 auftritt, weist dort demnach einen Wert von Null auf (β = 0°; vgl. Fig. 2A).

Dagegen verdeutlicht die Fig. 2B eine Inversion des Fußes 16, die mit einer Supination 36 einhergehen kann, woraus sich eine winkelige Anstellung des am Fuß 16 und/oder an einem Schuh (nicht gezeigt) verankerten unteren Teilabschnittes 12 ergibt, die durch Beweglichkeit im Gelenk 26 sowie durch entsprechende Gestaltung und Dimensionierung des Führungselementes 28 in der zeigten Weise eine Bewegungsfreiheit mit einem Grenzwinkel β_{Sup} für die Supination 36 zwischen 0° und etwa 7° (β_{Sup} = 0° ... 7°) erlaubt.

Weiterhin verdeutlicht die Fig. 2C ein Auswärtsdrehen oder eine Eversion des Fußes 16 durch eine Bewegung im Knöchelgelenk 24, die sich auch als Teil einer zu behandelnden Pronation des Fußes 16 manifestieren kann und deren Bewegungsrichtung durch einen mit der Bezugsziffer 40 gekennzeichneten Pfeil weg von der Medialseite 38 und hin zur Lateralseite 14 des Fußes 16 veranschaulicht wird. Um bei der erwähnten Überdehnung oder einem zu therapierenden Trauma des Bänderapparates im Fuß 16 und/oder im Bereich des Knöchelgelenks 24 die Pronation 40 (oder Eversion) zu begrenzen, kann die gezeigte Fußgelenk-Orthese 10 insbesondere eine Begrenzung des Schwenkwinkels β_{Pro} auf eine Größenordnung von ca. 10° bis 20° vorsehen, wobei dieser Pronationswinkel β_{Pro} vorzugsweise bei einem Ausgangswert von nicht mehr als etwa 15° liegen kann, der bedarfsweise durch Anpassung der Orthese 10 oder durch deren Austausch gegen eine modifizierte Orthese 10 mit anderen Dimensionierungen des Führungselementes 28 erhöht oder reduziert werden kann.

Da aus der orthopädischen Heilbehandlung normalerweise Winkelwerte als der Ausgangsstellung (β_{Pro} = 0°; vgl. Fig. 2A) in Eversionsrichtung in einer Größenordnung von etwa 15° (d.h. β_{Pro} 15°) vorgegeben sind, kann eine solchermaßen eingestellte Fußgelenk-Orthese 10 als Ausgangspunkt der Behandlung dienen und nach einiger Zeit und entsprechenden Heilungsfortschritten bedarfsweise gegen eine Orthese 10 mit größerem Grenzwinkel für β_{Pro} ausgetauscht werden.

Wie erwähnt, verdeutlicht die Fig. 2C eine Eversion oder Pronation 40 des Fußes 16, woraus sich eine Auswärtsdrehung und eine winkelige Anstellung des am Fuß 16 und/oder an einem Schuh (nicht gezeigt) verankerten unteren Teilabschnittes 12 der Orthese 10 ergibt, die durch die Beweglichkeit im Gelenk 26 sowie durch entsprechende Gestaltung und Dimensionierung des Führungselementes 28 in der zeigten Weise eine Bewegungsfreiheit mit einem Grenzwinkel β_{Pro} für die Eversion oder Pronation 40 zwischen 0° und etwa 15° (β_{Pro} = 0° ... 15°) erlaubt.

Es soll an dieser Stelle vorsorglich nochmals dasselbe betont sein, wie es schon weiter oben in Bezug auf die Figuren 1A bis 1C erwähnt wurde, dass die in den Figuren 2A bis 2C schematisch gezeigten Komponenten der Fußgelenk-Orthese 10, die in der beschriebenen Weise zusammenwirken, nicht zwingend als konkrete körperliche Bauteile in der zeichnerisch dargestellten Form zu verstehen sind, sondern dass sie als Wirkungselemente aufgefasst werden sollen, die bei der beschriebenen Positionierung der Orthese 10 an der Lateralseite 14 bzw. 20 des Fußes 16 bzw. des Unterschenkels 22 des Trägers die hier beschriebene Wirkung entfalten kann, insbesondere durch Begrenzung der Bewegungswinkel im Knöchelgelenk 24 bei den beschriebenen Schwenkbewegungen.

Die nachfolgend erläuterten Figuren 3A bis 5B zeigen allesamt verschiedene Ansichten und unterschiedliche Winkelstellungen einer konkret ausgestalteten, aber grundsätzlich ebenfalls beispielhaft zu verstehenden Ausführungsvariante der erfindungsgemäßen Fußgelenk-Orthese 10 sowie ihrer zusammenwirkenden Funktionselemente.

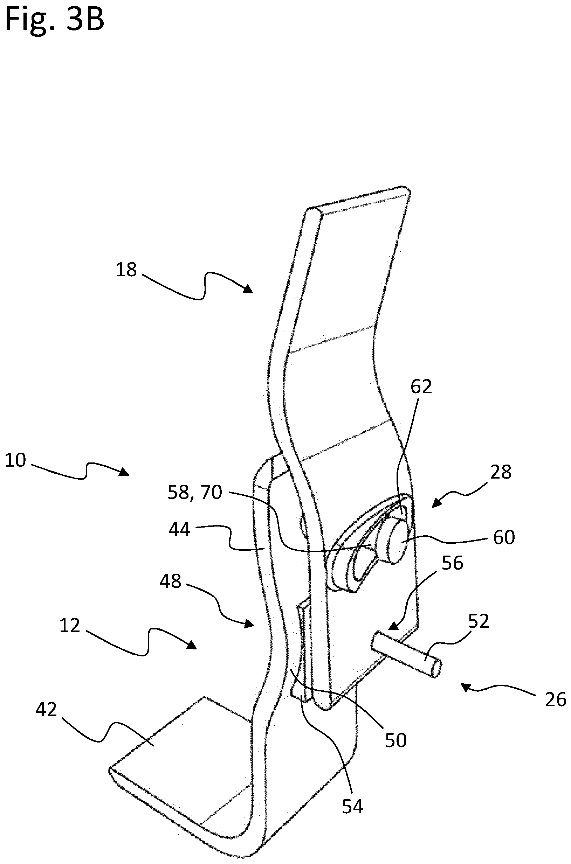

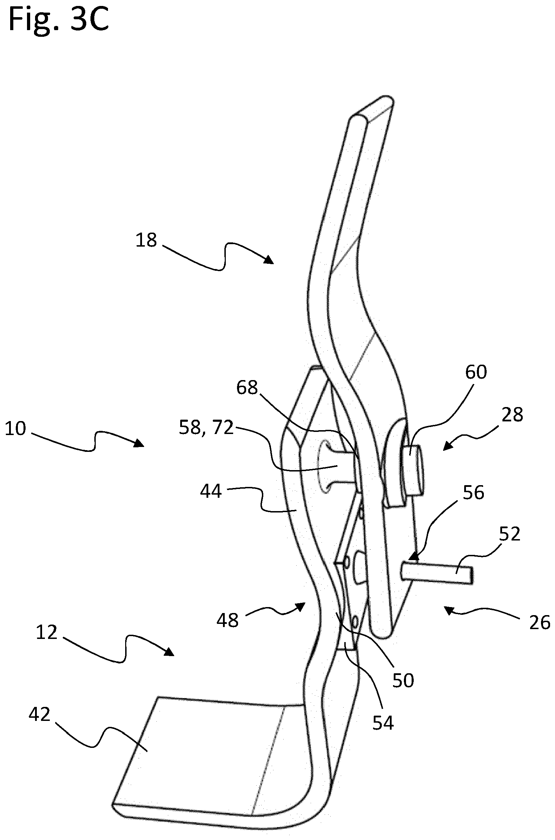

So zeigen die Figuren 3A, 3B und 3C jeweils schematische und perspektivische Ansichten auf eine denkbare Ausführungsvariante der erfindungsgemäßen Fußgelenk-Orthese 10. In den Figuren 3A bis 5B werden die oben bereits eingeführten Bezeichnungen sowie die jeweils zugehörigen Bezugsziffern verwendet. Die Figuren 3A bis 5B zeigen aber deutlich mehr Details, nämlich hinsichtlich der Ausgestaltung des Gelenks 26, das den unteren Teilabschnitt 12 gelenkig mit dem oberen Teilabschnitt 18 verbindet, sowie insbesondere hinsichtlich der Ausgestaltung des Führungselementes 28, das die Beweglichkeit der Teilabschnitte 12 und 18 zueinander definiert und ihre winkelige Anstellung in alle Richtungen innerhalb gewünschter und konstruktiv vorgegebener Grenzwinkel einschränkt.

Der untere Teilabschnitt 12 ist in der gezeigten Weise durch ein L-förmiges flaches Bauteil gebildet, dessen unterer Basisabschnitt 42 bspw. in einer Sohle eines Schuhs oder eines anderen geeigneten Fußbekleidungsstückes eingearbeitet und dort fixiert sein kann, was insbesondere einer festen räumlichen Zuordnung der Fußgelenk-Orthese 10 zum Fuß 16 (vgl. Figuren 1A bis 2C) eines Trägers oder Patienten dient. Wenn der Fuß 16 mit seiner unteren Sohlenseite auf dem Basisabschnitt 42 steht, so befindet sich ein ungefähr orthogonal zum horizontal angeordneten Basisabschnitt 42 stehender Anlageabschnitt 44 an der Lateralseite 14 des zu stabilisierenden Fußes 16 und liegt vorzugsweise dort an. Eine Fixierung des Anlageabschnittes 44 am Fuß ist normalerweise nicht vorgesehen, da seine Lage durch den auf dem Basisabschnitt 42 stehenden und diesen belastenden Fuß fixiert und räumlich definiert ist.

Wie es die Figuren 3A, 3B und 3C erkennen lassen, gehen der untere horizontale Basisabschnitt 42 des unteren Teilabschnittes 12 und der sich ungefähr im 90°-Winkel in vertikale Richtung daran anschließende Anlageabschnitt 44 mit sanfter Verrundung 46 ineinander über, wobei die Verrundung 46 sinnvollerweise der typischen Kontur der Lateralseite 14 des Fußes 16 folgt, was sowohl den Tragekomfort der Orthese 10 als auch deren Sitz am Fuß 16 begünstigt.

Der in etwa vertikal verlaufende Anlageabschnitt 44 weist eine dem Fuß 16 im Höhe des Knöchelbereiches zugewandte konkave Einwölbung 48 auf, um der Fußkontur besser zu folgen und um insbesondere nach Möglichkeit Druckstellen und/oder eine schlechte Passform der Orthese 10 zu vermeiden, denn der gesamte untere Teilabschnitt 12 liegt weitgehend flächig am Fuß 16 des Trägers an und verläuft hierbei von der Sohle bis knapp oberhalb des Knöchelbereiches, wo der in etwa vertikal stehende Anlageabschnitt 44 endet.

Im gezeigten Ausführungsbeispiel ist der untere Teilabschnitt 12 als flacher Streifen ausgebildet, der eine deutlich größere Breite als Dicke aufweist. Die Dicke oder Materialstärke des Streifens kann so gering wie möglich ausfallen, um den Tragekomfort und die Integrierbarkeit in einen Schuh oder Stiefel zu verbessern, muss jedoch ausreichend stabil sein, um zu verhindern, dass sich der untere Teilabschnitt 12 beim Tragen unter den Fußbewegungen zu stark verformen kann. Sollte dies der Fall sein, so könnten die angestrebten mechanischen Funktionen, die im Wesentlichen in der Begrenzung der Bewegungswinkel zwischen den gelenkig miteinander verbundenen Teilabschnitten 12 und 18 liegen, beeinträchtigt sein. Die Breite des Streifens, der den unteren Teilabschnitt 12 bildet, richtet sich in erster Linie an der gewünschten Aufstandsfläche des horizontalen Basisabschnittes 42, an der gewünschten Anlagefläche des sich in vertikale Richtung über die Verrundung 46 an den Basisabschnitt 42 anschließenden Anlageabschnittes 44 sowie an den im unteren Teilabschnitt 12 unterzubringenden Komponenten und Wirkelementen des Gelenks 26 sowie des Führungselementes 28.

Eine sinnvolle Breite des unteren Teilabschnittes 12 kann bspw. bei ca. zwei bis fünf Zentimetern liegen, während eine sinnvolle Materialstärke vom verwendeten Material abhängt. Ein hochfestes Fasermaterial wie bspw. CFK oder auch GFK ermöglicht einen relativ dünn ausgeführten Streifen mit einer Materialstärke von deutlich unter zwei Millimetern. Gleiches gilt für ein hochfestes und ausreichend flexibles Metallmaterial wie bspw. eine geeignete Aluminium- oder Titanlegierung, die sehr dünne Materialstärken von weniger als einem Millimeter erlaubt.

An der vom Fuß 16 abgewandten Außenseite des Anlageabschnittes 44, etwa in Höhe des Knöchelbereiches des Fußes und damit an der gegenüberliegenden Seite der konkaven Einwölbung 48, befindet sich der dem unteren Teilabschnitt 12 zugeordnete Bereich des Gelenks 26, der die beiden Teilabschnitte 12 und 18 gelenkig miteinander verbindet.

Wie oben erwähnt, könnte das Gelenk 26 wahlweise als Kugelgelenk ausgebildet sein, so dass der untere Teilabschnitt 12 z.B. mit einer Gelenkpfanne auszustatten wäre, in der sich eine mit dem oberen Teilabschnitt 18 verbundene Gelenkkugel weitgehend spielfrei drehen könnte, so dass eine allseitige Verdrehung um den Drehpunkt eines solchen Kugelgelenks ermöglicht wäre.

Stattdessen befindet sich auf der konvex ausgewölbten Seite des Anlageabschnittes 44 ein senkrecht nach außen weisender Stift 52, der an der konvexen Auswölbung 50 des Anlageabschnittes 44 verankert ist und somit in etwa horizontal verläuft. Der Stift 52 ragt mittig aus einem Sockel 54, der die konvexe Auswölbung 50 fortsetzt und dem Stift 52 solchermaßen eine stabile Verankerung bietet, so dass er gewissen mechanischen Belastungen standhalten kann, die bei der Verwendung der Fußgelenk-Orthese 10 auftreten können.

Der Stift 52 führt den mit passender Bohrung 56 ausgestatteten oberen Teilabschnitt 18, wobei die Bohrung 56 einen größeren Bohrungsdurchmesser aufweist als der Außendurchmesser des Stiftes 52, so dass ein ausreichendes Spiel für Verschwenkungen und Verkippungen des am Stift 52 als Gelenkachse aufgehängten und geführten oberen Teilabschnittes 18 gegeben ist. Außerdem erlaubt es die lose Längsführung der Bohrung 56 auf dem Stift 52, dass der Abstand des oberen Teilabschnittes 18 im Bereich dieser gelenkigen Lagerung (Gelenk 26) zum unteren Teilabschnitt 12 in gewissen Grenzen variabel bleibt.

Der maximale Abstand der beiden Abschnitte 12 und 18 zueinander ist jedoch durch die nachfolgend beschriebene Ausgestaltung und Funktionsweise des mit dem Gelenk 26 zusammenwirkenden und vom Gelenk 26 beabstandeten Führungselementes 28 vorgegeben, während sich der minimale Abstand beim vollständigen Aufschieben des oberen Teilabschnittes 18 mit seiner am Stift 52 geführten Bohrung 56 auf den Sockel 54 einstellt.

Wie es die Figuren 3A, 3B und 3C weiterhin erkennen lassen, umfasst die Fußgelenk-Orthese 10 neben dem ausführlich in seinen einzelnen Elementen erläuterten unteren Teilabschnitt 12 den über das Gelenk 26 und das Führungselement 28 damit verbundenen oberen Teilabschnitt 18, der an der Lateralseite 20 des Unterschenkels 22 des Trägers anliegt und insbesondere dort fixiert ist, und zwar in einem Bereich deutlich oberhalb des Knöchelgelenkes 24 des Fußes 16.

Das Führungselement 28 ist proximal beabstandet zum Gelenk 26 angeordnet, womit eine Positionierung oberhalb des Knöchelgelenks 24 und näher am oberen Teilabschnitt 18 bzw. integriert im oberen Teilabschnitt 18 gemeint ist. Das Führungselement 28, das neben dem Gelenk 26 eine weitere Verbindung zwischen unterem Teilabschnitt 12 und oberem Teilabschnitt 18 herstellt, sorgt im Wesentlichen für die gewünschte Winkel- und Wegbegrenzung der Beweglichkeit und Verschwenkbarkeit der oberen und unteren Teilabschnitte 12 und 18 gegeneinander.

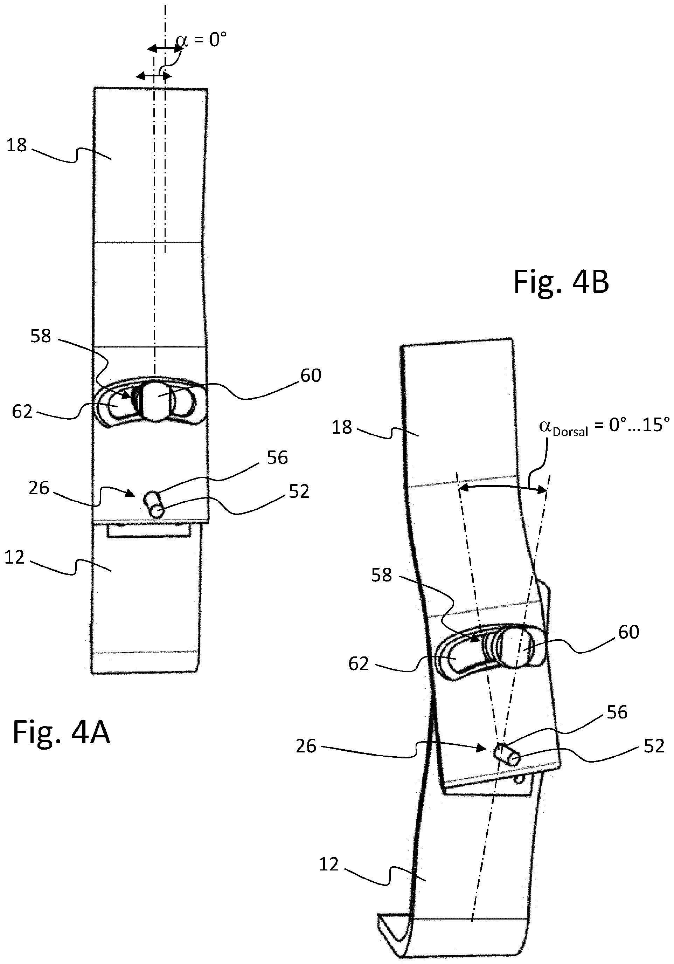

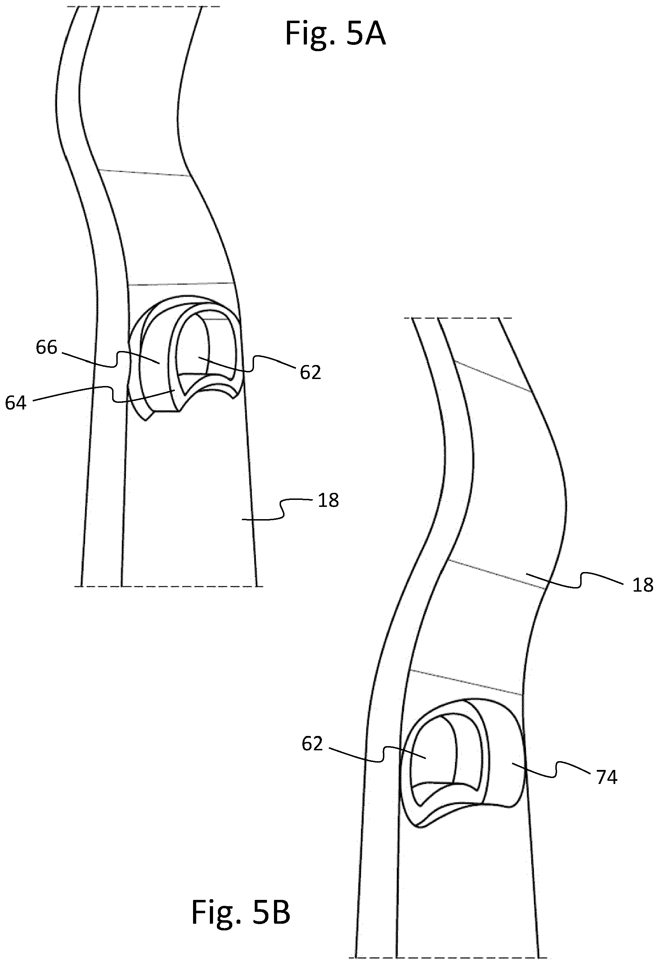

Das Führungselement 28, dessen Wirkungsweise hinsichtlich der zu begrenzenden Schwenkwinkel bei einer Plantarflexion und einer Dorsalextension bereits in seinen Grundsätzen anhand der Figuren 1 B und 1C erläutert wurde, wird nachfolgend unter Bezugnahme auf die Figuren 4A bis 4D im Detail beschrieben, während sich die schon grundsätzlich anhand der Figuren 2B und 2C erläuterte Winkelbegrenzung bei einer Inversion und/oder Eversion des Fußes besser unter Bezugnahme auf die Figuren 5A und 5B im Detail zeigen lässt.

Das Führungselement 28 ist gebildet durch einen starr am oberen Bereich des Anlageabschnittes 44 verankerten zylindrischen Zapfen 58 mit pilzförmiger Verbreiterung 60 am freien Ende, der in einer nutartigen Führungskulisse 62 des oberen Teilabschnittes 18 geführt ist und dort entlanggleiten kann, wobei die zum Zapfen 58 weisende Stirnseite der pilzförmigen Verbreiterung 60 auf einem konturierten und über eine definierte Schrägrampe 64 verfügenden erhöhten äußeren Rand 66 der Führungskulisse 62 geführt und damit in seiner Bewegungsfreiheit eingeschränkt ist.

Außerdem lässt die Fig. 3C eine beabstandet zur endseitigen pilzförmigen Verbreiterung 60 befindliche weitere scheibenförmige Verbreiterung 68 erkennen, die den Zapfen 58 seiner Länge nach in etwa mittig unterteilt, wodurch sich ausschließlich ein zwischen den beiden Verbreiterungen 60 und 68 befindlicher äußerer zylindrischer Abschnitt 70 (vgl. Fig. 3B) des Zapfens 58 innerhalb der Führungskulisse 62 bewegen kann, während ein zwischen Anlageabschnitt 44 und der weiteren scheibenförmigen Verbreiterung 68 liegender innerer Abschnitt 72 des Zapfens 58 nicht mit dem oberen Teilabschnitt 18 oder der darin befindlichen Führungskulisse 62 in Kontakt kommt. Der innere Abschnitt 72 dient lediglich der festen Verankerung des Zapfens 58 im oberhalb des Sockels 54 mit dem Stift 52 befindlichen oberen Bereich des Anlageabschnittes 44 des unteren Teilabschnittes 12, während die Länge des Zapfens 58 und die Positionen der daran angeordneten Verbreiterungen 60 und 68 im Zusammenhang mit der Konturierung des die Schrägrampe 64 bildenden umgebenden Randes 66 der Führungskulisse die Winkelbegrenzungen bei einer Pronation 40 oder Supination 36 des Fußes 16 definieren.

So definiert die den gesamten umgebenden Rand 66 der Führungskulisse 62 bildende Schrägrampe 64, die die äußere pilzförmige Verbreiterung 60 am Zapfen 58 führt, im Wesentlichen die maximalen Supinationswinkel β_{Sup}, die durch die Kontur, den Verlauf und die Höhe der Schrägrampe 64 eingeschränkt sind. Die mögliche und sinnvolle Gestaltung der Schrägrampe 64 wird weiter unten unter Bezugnahme auf die Fig. 5A näher erläutert.

Dagegen werden die maximalen Pronationswinkel β_{Pro} durch eine Kontur einer die Führungskulisse 62 zur Innenseite umgebenden Randgestaltung definiert, die eine Führung für die weitere scheibenförmige Verbreiterung 68 des Zapfens 58 bildet, wobei diese innere Randgestaltung der Führungskulisse 62 ebenfalls weiter unten unter Bezugnahme auf die Fig. 5B näher erläutert wird.

Wie es die Figuren 4A bis 4D verdeutlichen, sorgt das Zusammenspiel des bei am Fuß 16 getragener Orthese 10 ungefähr in Höhe des Knöchelgelenks 24 (vgl. Fig. 1A) befindlichen Gelenks 26 mit dem in proximaler Richtung beabstandet dazu angeordneten Führungselement 28 für die gewünschten Winkelbegrenzungen sowohl bei einer Plantarflexion 34 (vgl. Fig. 1C) als auch bei einer Dorsalextension 32 (vgl. Fig. 1B). Die in den Figuren 4A bis 4D jeweils von der Seite gezeichnete und an den Lateralseiten 14 und 20 des linken Fußes 16 bzw. Unterschenkels 22 getragene Fußgelenk-Orthese 10 verdeutlicht verschiedene Fußpositionen und entsprechende Winkelstellungen des oberen Teilabschnittes 18 zum unteren Teilabschnitt 12, wobei die maximalen Schwenkwinkel α_{Dorsal} für die Dorsalextension 32 sowie α_{Plantar} für die Plantarflexion 34 jeweils durch die Dimensionierung der mit dem Zapfen 58 zusammenwirkenden Führungskulisse 62 definiert und begrenzt sind.

Die Seitenansicht der Fig. 4A zeigt die Fußgelenk-Orthese 10 mit annähernd fluchtenden unteren und oberen Abschnitten 12 und 18, so dass hierbei der Winkel α in etwa den Wert Null hat. Die Fußstellung entspricht hierbei derjenigen entsprechend Fig. 1A, bei welcher der Fuß 16 weder nach oben gestreckt noch nach unten gebeugt ist. Der zylindrische Zapfen 58 befindet sich hierbei ungefähr mittig in der länglichen und einem leicht gekrümmten Verlauf folgenden Führungskulisse 62, wobei die stirnseitige pilzartige Verbreiterung 60 des Zapfens 58 auf dem weiter unten erläuterten erhöhten äußeren Rand 66 mit seiner Schrägrampe 64 (vgl. Fig. 5A) aufliegt. Da jedoch der zwischen den beiden Verbreiterungen 68 und 60 befindliche äußere zylindrische Abschnitt 70 des Zapfens 58 mit geringem Spiel in der Führungskulisse 62 gleiten kann, gibt diese die Beweglichkeit der Teilabschnitte 12 und 18 zueinander vor.

Die weitere Seitenansicht der Fig. 4B verdeutlicht eine erste Schwenkposition des oberen Teilabschnittes 18 gegen den unteren Teilabschnitt 12 um das untere Gelenk 26. Da sich die Bohrung 56 des oberen Teilabschnittes 18 auf dem Stift 52 des unteren Teilabschnittes 12 befindet, bildet diese Passung ein Drehgelenk 26 mit einer Drehachse, die mittig durch den Stift 52 bzw. die Bohrung 56 verläuft, so dass die Verschwenkung der beiden Teilabschnitte 12 und 18 zueinander in der gezeigten Weise erfolgen kann. Die Krümmung der länglichen Führungskulisse 62 hat demzufolge ihren Mittelpunkt in etwa in dieser durch den Stift 52 bzw. durch die Bohrung 56 definierten Drehachse.

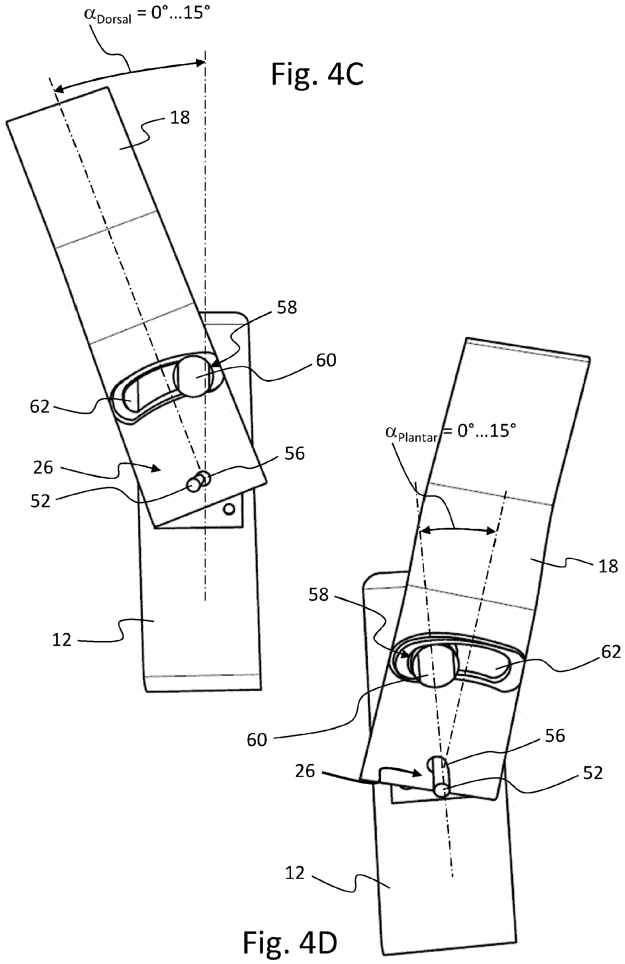

Während die Fig. 4B eine Dorsalextension 32 mit einem Dorsalwinkel α_{Dorsal} von weniger als den maximal möglichen 15° verdeutlicht, zeigt die Seitenansicht der Fig. 4C den am Rand der Führungskulisse 62 anliegenden Zapfen 58, wodurch der maximale Dorsalwinkel α_{Dorsal} von ca. 15° erreicht ist.

Dagegen verdeutlicht die Fig. 4D eine Plantarflexion 34 des Fußes gemäß Fig. 1C, bei der allerdings der Plantarwinkel α_{Plantar} etwas weniger als 15° beträgt, da der Zapfen 58 den gegenüberliegenden Rand der länglichen Führungskulisse 62 und damit den mechanischen Anschlag für die Begrenzung des Plantarwinkels α_{Plantar} noch nicht erreicht hat.

Auf eine Darstellung des Fußes 16, des Unterschenkels 22 und der Bandage 30 wurde in den Figuren 4A bis 4D, ebenso wie in den Figuren 3A bis 3C, jeweils verzichtet.

Schließlich verdeutlicht die Fig. 5A eine mögliche Konturierung des erhöhten äußeren Randes 66 der Führungskulisse 62, die hinsichtlich der Anlageführung für die pilzförmige äußere Verbreiterung 60, die den Zapfen 58 endseitig abschließt, für die Winkelbegrenzungen bei einer Inversion oder Supination des Fußes 16 sorgen können. Bei einem stärker erhöhten Rand 66 und entsprechend steilerer Schrägrampe 64 verbleibt ein geringerer axialer Bewegungsspielraum für den zwischen den Verbreiterungen 60 und 68 befindlichen äußeren Zapfenabschnitt 70, wodurch auch der Supinationswinkel β_{Sup} stärker begrenzt wird als bei einem flacher gestalteten Rand 66 der Führungskulisse 62.

Außerdem verdeutlicht die Fig. 5B eine mögliche Konturierung des als zusätzliche Führungsnut 74 gestalteten inneren Randes der Führungskulisse 62, die eine Breite aufweist, welche dem Außendurchmesser der weiteren scheibenförmigen Verbreiterung 68 des Zapfens 58 entspricht und diese Verbreiterung 68 somit parallel zur Führung des Zapfens 58 innerhalb der Öffnung der Führungskulisse 62 führen kann. Die Tiefe der zusätzlichen Führungsnut 74 und damit die verbleibende Stärke der Führungskulisse 62 definiert wiederum den axialen Bewegungsspielraum für den zwischen den Verbreiterungen 60 und 68 befindlichen äußeren Zapfenabschnitt 70, wobei der Pronationswinkel β_{Pro} durch die Anlage der Verbreiterung 68 im Grund der Führungsnut 74 begrenzt wird, denn bei einer weniger starken Vertiefung der Führungsnut 74 verbleibt ein geringerer axialer Bewegungsspielraum für den zwischen den Verbreiterungen 60 und 68 befindlichen äußeren Zapfenabschnitt 70, wodurch auch der Pronationswinkel β_{Pro} stärker begrenzt wird als bei einer tiefer ausgeführten Führungsnut 74 an der in Fig. 5B gezeigten Rückseite der Führungskulisse 62.

Die Länge der Führungskulisse 62, die Höhe des äußeren Randes 66 sowie die Tiefe der die Führungskulisse 62 an der Innenseite umgebenden zusätzlichen Führungsnut 74 definieren den Bewegungsspielraum bei den in den Figuren 1A bis 2C verdeutlichten Fußbewegungen, wenn die Fußgelenk-Orthese 10 getragen wird.

An dieser Stelle ist es sinnvoll zu betonen, dass neben den oben aufgeführten und sich auf die Figuren 1A bis 5B beziehenden Ausführungsbeispielen der Erfindung weitere Ausführungsvarianten der erfindungsgemäßen Fußgelenk-Orthese 10 möglich sind. Bei einer dieser weiteren Ausführungsvarianten kann eine alternative Montagevariante des unteren Teilabschnittes 12 an der Lateralseite 14 des zu stabilisierenden Fußes 16 vorgesehen sein. Neben der oben dargestellten äußeren Fixierung und/oder Montage des unteren Teilabschnittes 12 an der Außenseite oder Lateralseite 14 des Fußes 16 kommt wahlweise auch eine Eingriffsmöglichkeit in den menschlichen Organismus in Frage, etwa durch eine Montage des unteren Teilabschnittes 12 am Knochenapparat (nicht gezeigt) des betroffenen Menschen mittels eines operativen und chirurgischen Eingriffes. Eine solche Montage am Knochenapparat kann bspw. durch Verkleben, durch Verschrauben, durch Verklammern oder durch Einfügen einer mehrteiligen Draht- und/oder Seilstruktur erfolgen, wobei eine solche mehrteilige Draht- und/oder Seilstruktur wiederum verklebt, verschraubt, verknotet, verklammert oder auf sonstige Weise mit unterschiedlichen, sinnvollerweise voneinander beabstandeten Knochenteilen des Fußes 16 verbunden werden kann.

Auf diese Weise kann eine stabile Verankerung des unteren Teilabschnittes 12 an der Lateralseite 14 des zu stabilisierenden Fußes 16 gewährleistet werden, die ein definiertes Widerlager für das spielbehaftete Gelenk 26 liefern kann.

Bei einer weiteren Ausführungsvarianten kann zudem in einer alternativen Montagevariante auch der obere Teilabschnitt 18 der Lateralseite 20 des Unterschenkels 22 oberhalb des Knöchelgelenks 24 des zu stabilisierenden Fußes 16 zugeordnet werden, indem ein Eingriff in den menschlichen Organismus gewählt wird, etwa durch eine Montage des oberen Teilabschnittes 18 am Knochenapparat des Unterschenkels 22 des betroffenen Menschen (nicht gezeigt) mittels eines operativen und chirurgischen Eingriffes. Eine solche Montage am Knochenapparat kann bspw. durch Verkleben, durch Verschrauben, durch Verklammern oder durch Einfügen einer mehrteiligen Draht- und/oder Seilstruktur erfolgen, wobei eine solche mehrteilige Draht- und/oder Seilstruktur wiederum verklebt, verschraubt, verknotet, verklammert oder auf sonstige Weise mit unterschiedlichen, sinnvollerweise voneinander beabstandeten Knochenabschnitten des Schienbeins und/oder des Wadenbeins des Unterschenkels 22 verbunden werden kann.

Im Ergebnis wird auch auf diese Weise eine stabile Verankerung des oberen Teilabschnittes 18 an der Lateralseite 20 des Unterschenkels 22 oberhalb des zu stabilisierenden Fußes 12 geschaffen, wodurch wiederum ein definiertes Widerlager für das spielbehaftete Gelenk 26 gegeben ist.

Beide Varianten können wahlweise in beliebiger Weise miteinander kombiniert werden, so etwa eine Verankerung des unteren Teilabschnittes 12 sowie des oberen Teilabschnittes 18 am jeweiligen Knochenapparat (des Fußes 12 bzw. des Unterschenkels 22), so dass hierbei sinnvollerweise auch das spielbehaftete Gelenk 26 unterhalb der Haut platziert wird. Wahlweise kann das spielbehaftete Gelenk 26 jedoch aus Platzgründen teilweise oder vollständig außerhalb des menschlichen Organismus positioniert sein, so dass gelenknahe Abschnitte des unteren sowie des oberen Teilabschnittes 12 bzw. 18 jeweils die Haut durchdringen können.

Wahlweise kommt auch eine Verankerung des unteren Teilabschnittes 12 am jeweiligen Knochenapparat in Frage, während der obere Teilabschnitt 18 ohne operative oder chirurgische Eingriffe an der Außenseite oder Lateralseite 20 des Unterschenkels 22 angelegt oder fixiert werden kann, entweder temporär oder dauerhaft. Sinnvollerweise wird bei dieser Variante auch das spielbehaftete Gelenk 26 außerhalb der Haut platziert, so dass lediglich ein gelenknaher Abschnitt des unteren Teilabschnittes 12 die Haut durchdringt.

Ebenso denkbar ist eine weitere Variante, bei der lediglich der obere Teilabschnitt 18 am jeweiligen Knochenapparat des betroffenen Unterschenkels 22 verankert wird, während der untere Teilabschnitt 12 ohne operative oder chirurgische Eingriffe an der Außenseite oder Lateralseite 14 des Fußes 12 angelegt oder fixiert werden kann, entweder temporär oder dauerhaft. Sinnvollerweise wird bei dieser Variante auch das spielbehaftete Gelenk 26 außerhalb der Haut platziert, so dass lediglich ein gelenknaher Abschnitt des oberen Teilabschnittes 18 die Haut zu durchdringen hat.

Da sich das Führungselement 28 in aller Regel und bei den meisten sinnvollen konstruktiven Ausgestaltungen der erfindungsgemäßen Fußgelenk-Orthese 10 in unmittelbarer Nähe zum spielbehafteten Gelenk 26 befindet, ist im Einzelfall zu entscheiden, ob bei einer Montage des oberen und/oder des unteren Teilabschnittes 18 und/oder 12 mittels operativen oder chirurgischen Eingriffes am jeweiligen Knochenapparat auch das Führungselement 28 gemeinsam mit dem Gelenk 26 (teilweise oder abschnittsweise) unterhalb der Haut oder (teilweise oder abschnittsweise) außerhalb des betroffenen menschlichen Organismus platziert werden kann.

Folgendes sei als ergänzender Hinweis zu den vorstehenden Ausführungen gegeben. Wenn auch im Zusammenhang mit den in den Figuren gezeigten Ausführungsvarianten der Fußgelenk-Orthese 10 und deren vorstehenden Beschreibungen oftmals oder auch generell von "schematischen" Darstellungen und Ansichten die Rede ist, so ist damit keineswegs gemeint, dass die Figurendarstellungen und deren Beschreibung hinsichtlich der Offenbarung der Erfindung bzw. der erfindungsgemäßen Orthese 10 von untergeordneter Bedeutung sein sollen. Der Fachmann ist durchaus in der Lage, aus den schematisch und abstrakt gezeichneten Darstellungen genug an Informationen zu entnehmen, die ihm das Verständnis der Erfindung erleichtern, ohne dass er etwa aus den gezeichneten und möglicherweise nicht exakt maßstabsgerechten Größenverhältnissen von Teilen der Orthese 10, deren Einzelheiten oder anderer gezeichneter Elemente in irgendeiner Weise in seinem Verständnis beeinträchtigt wäre. Die Figuren ermöglichen es dem Fachmann als Leser vielmehr, anhand der konkreter erläuterten Umsetzungen des konkreter erläuterten Aufbaus der erfindungsgemäßen Orthese 10 ein besseres Verständnis für den in den Ansprüchen sowie im allgemeinen Teil der Beschreibung zumindest hinsichtlich einiger Aspekte allgemeiner und/oder abstrakter formulierten Erfindungsgedanken abzuleiten.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 10: Orthese, Fußgelenk-Orthese
- 12: unterer Teilabschnitt
- 14: Lateralseite (Fuß)
- 16: Fuß
- 18: oberer Teilabschnitt
- 20: Lateralseite (Unterschenkel)
- 22: Unterschenkel
- 24: Knöchelgelenk, Fußgelenk
- 26: Gelenk (Orthese)
- 28: Führungselement
- 30: Band, Bandage
- 32: Dorsalextension
- 34: Plantarflexion
- 36: Supination
- 38: Medialseite (Fuß)
- 40: Pronation
- 42: Basisabschnitt
- 44: Anlageabschnitt
- 46: Verrundung
- 48: konkave Einwölbung
- 50: konvexe Auswölbung
- 52: Stift
- 54: Sockel
- 56: Bohrung
- 58: Zapfen, zylindrischer Zapfen
- 60: Verbreiterung, pilzförmige Verbreiterung
- 62: Führungskulisse
- 64: Schrägrampe
- 66: Rand, erhöhter äußerer Rand
- 68: Verbreiterung, weitere scheibenförmige Verbreiterung
- 70: äußerer zylindrischer Abschnitt (Zapfen)
- 72: innerer Abschnitt (Zapfen)
- 74: zusätzliche Führungsnut (Führungskulisse)
- α: Schwenkwinkel
- α_{Dorsal}: Dorsalwinkel
- α_{Plantar}: Plantarwinkel
- β: Schwenkwinkel
- β_{Sup}: Supinationswinkel
- β_{Pro}: Pronationswinkel

## Patentansprüche

1. Fußgelenk-Orthese (10) zur Begrenzung eines Bewegungsumfangs im Fußknöchelgelenk (24) eines menschlichen Fußes (16) und/oder zur mechanischen Stabilisierung des Bänderapparats im Fußknöchelgelenk (24), welche Orthese (10) mindestens zwei gelenkig miteinander verbundene Teilabschnitte (12, 18) umfasst,
- wobei ein unterer Teilabschnitt (12) einer Lateralseite (14) des zu stabilisierenden Fußes (16) zuordenbar ist, und
- wobei ein oberer Teilabschnitt (18) einer Lateralseite (20) des Unterschenkels (22) oberhalb des Knöchelgelenks (24) des Fußes (16) zuordenbar ist,
- wobei der obere Teilabschnitt (18) und der untere Teilabschnitt (12) mittels eines in Nähe des Knöchelgelenks (24) befindlichen, spielbehafteten Gelenks (26) beweglich miteinander verbunden sind, und
- wobei proximal beabstandet zum Gelenk (26) ein Führungselement (28) zur Winkel- und/oder Wegbegrenzung der Beweglichkeit und Verschwenkbarkeit der oberen und unteren Teilabschnitte (18, 12) gegeneinander angeordnet ist, welches Führungselement (28) jeweils die maximalen Schwenkwinkel (α, β) sowohl
(i) bei einer Dorsalextension (32) als auch
(ii) bei einer Plantarflexion (34) auf definierte Grenzwinkel (α_{Dorsal}, α_{Plantar}) einschränkt bzw. begrenzt,
**dadurch gekennzeichnet, dass** das Führungselement (28) durch eine Führungskulisse (62) und einem darin insbesondere leicht spielbehaftet geführten und verschiebbaren und/oder winkelig anstellbaren Zapfen (58) gebildet ist.

2. Fußgelenk-Orthese nach Anspruch 1, bei welcher die Führungskulisse (62) durch eine bogenförmige Nut gebildet ist, die den Zapfen (58) führt.

3. Fußgelenk-Orthese nach Anspruch 1 oder 2, bei welcher die Führungskulisse (62) einen konturierten äußeren Rand (66) aufweist, wobei eine Randkontur den Anstellwinkel (β) zwischen den gelenkig miteinander verbundenen Teilabschnitten (12 und 18) definiert und begrenzt.

4. Fußgelenk-Orthese nach einem der Ansprüche 1 bis 3, bei welcher der in der Führungskulisse (62) geführte Zapfen (58) endseitig einen vergrößerten Kopf (60) aufweist, der mit der korrespondierenden Randkontur (66) der Führungskulisse (62) zusammenwirkt und für die Begrenzung der Anstellwinkel (β) zwischen den gelenkig miteinander verbundenen Teilabschnitten (12 und 18) sorgt.

5. Fußgelenk-Orthese nach einem der Ansprüche 1 bis 4, bei welcher der in der Führungskulisse (62) geführte Zapfen (58) einen verbreiterten Sockel oder eine Verbreiterung (68) aufweist, der/die mit einer Rückseite der Führungskulisse (62), insbesondere mit einer rückseitigen Randkontur (74) der Führungskulisse (62) zusammenwirkt und für die Begrenzung der Anstellwinkel (β) zwischen den gelenkig miteinander verbundenen Teilabschnitten (12 und 18) sorgt.

6. Fußgelenk-Orthese (10) nach einem der Ansprüche 1 bis 5, bei der das Führungselement (28) zusätzlich
(iii) einen Supinationswinkel (β_{Sup}) sowie einen Pronationswinkel (β_{Pro}) im Fußgelenk (24) durch Einschränkung der Anstellwinkel (β) zwischen oberem und unterem Teilabschnitt (18, 12) begrenzt.

7. Fußgelenk-Orthese (10) nach einem der Ansprüche 1 bis 6, die der äußeren mechanischen Stabilisierung des Bänderapparats im Fußknöchelgelenk (24) dient, wobei der untere Teilabschnitt (12) an einer Lateralseite (14) des zu stabilisierenden Fußes (16) anlegbar und/oder dort fixierbar ist, und/oder wobei der obere Teilabschnitt (18) an einer Lateralseite (20) des Unterschenkels (22) oberhalb des Knöchelgelenks (24) des Fußes (16) anlegbar und/oder dort fixierbar ist.

8. Fußgelenk-Orthese nach einem der Ansprüche 1 bis 7, bei welcher der obere Teilabschnitt (18) durch ein flaches Formteil gebildet ist, wobei der obere Teilabschnitt (18) mittels einer Bandage (30) am Unterschenkel (22) des Trägers festlegbar ist.

9. Fußgelenk-Orthese nach einem der Ansprüche 1 bis 8, bei welcher der untere Teilabschnitt (12) durch ein flaches und an den Fuß (16) angeformtes Formteil gebildet ist, das am Fuß (16) und/oder an einer Fußoberbekleidung wie einem Schuh oder Stiefel fixiert oder fixierbar ist.

10. Fußgelenk-Orthese nach Anspruch 9, bei welcher der untere Teilabschnitt (12) mit einem abgewinkelten Basisabschnitt (42) die Fußsohle des Trägers untergreift.

11. Fußgelenk-Orthese nach einem der Ansprüche 1 bis 10, bei welcher das Gelenk (26) zur beweglichen Verbindung des unteren und des oberen Teilabschnittes (18, 12) durch ein Kugelgelenk gebildet ist.

12. Fußgelenk-Orthese nach Anspruch 11, bei welcher das Gelenk (26) oder Kugelgelenk einen zusätzlich Freiheitsgrad aufweist, indem der Abstand der beiden Teilabschnitte (12 und 18) senkrecht zu ihren zueinander weisenden Oberflächen innerhalb eines definierten Spielraums variabel ist.

13. Fußgelenk-Orthese nach einem der Ansprüche 1 bis 12, bei welcher zwischen dem Gelenk (26) und dem Führungselement (28) bzw. dessen Führungskulisse (62) ein definierter Mindestabstand von wenigstens 20 Millimetern liegt.

## Claims

1. An ankle orthosis (10) for limiting a range of motion in the ankle joint (24) of a human foot (16) and/or for mechanically stabilizing the ligamentous apparatus in the ankle joint (24), which orthosis (10) comprises at least two partial sections (12, 18) connected to each other in an articulated manner,
- wherein a lower partial section (12) is assignable to a lateral side (14) of the foot (16) to be stabilised, and
- wherein an upper partial section (18) is assignable to a lateral side (20) of the lower leg (22) above the ankle joint (24) of the foot (16),
- wherein the upper partial section (18) and the lower partial section (12) are movably connected to each other by means of a joint (26) with clearance, which joint (26) is situated in the vicinity of the ankle joint (24), and
- wherein, arranged proximally spaced apart from the joint (26), there is a guide element (28) for angular limitation and/or path limitation of the mobility and pivotability of the upper and lower partial sections (18, 12) relative to each other, which guide element (28) in each case restricts or limits the maximum pivot angles (α, β), both
(i) in the case of dorsal extension (32) and
(ii) in the case of plantar flexion (34), to defined critical angles (α_{Dorsal}, α_{Plantar}), **characterised in that** the guide element (28) is formed by a guide slot (62) and a pin (58) guided therein, in particular with slight clearance, which pin (58) is shiftable and/or angularly adjustable.

2. The ankle orthosis according to claim 1, in which the guide slot (62) is formed by an arc-shaped groove, which guides the pin (58).

3. The ankle orthosis according to claim 1 or 2, in which the guide slot (62) has a contoured outer edge (66), wherein an edge contour defines and limits the adjustment angle (β) between the partial sections (12 and 18), which are articulately connected to each other.

4. The ankle orthosis according to one of the claims 1 to 3, in which the pin (58), which is guided in the guide slot (62), has an enlarged head (60) at the end, which head (60) interacts with the corresponding edge contour (66) of the guide slot (62) and provides for limiting the adjustment angles (β) between the partial sections (12 and 18), which are articulately connected to each other.

5. The ankle orthosis according to one of the claims 1 to 4, in which the pin (58), which is guided in the guide slot (62), has widened base or a widening (68), which interacts with a back side of the guide slot (62), in particular with a back side edge contour (74) of the guide slot (62), and which provides for limiting the adjustment angles (β) between the partial sections (12 and 18), which are articulately connected to each other.

6. The ankle orthosis (10) according to one of the claims 1 to 5, in which the guide element (28) additionally
(iii) limits a supination angle (β_{Sup}) and a pronation angle (β_{Pro}) in the ankle joint (24) by restricting the adjustment angles (β) between the upper partial section (18) and the lower partial section (12).

7. The ankle orthosis (10) according to one of the claims 1 to 6, which serves for the external mechanical stabilising of the ligamentous apparatus in the ankle joint (24), wherein the lower partial section (12) is bringable into contact with and/or fixable to a lateral side (14) of the foot (16) to be stabilised, and/or wherein the upper partial section (18) is bringable into contact with and/or fixable to a lateral side (20) of the lower leg (22) above the ankle joint (24) of the foot (16).

8. The ankle orthosis according to one of the claims 1 to 7, in which the upper partial section (18) is formed by a flat formed part, wherein the upper partial section (18) is securable to the lower leg (22) of the wearer by means of a brace (30).

9. The ankle orthosis according to one of the claims 1 to 8, in which the lower partial section (12) is formed by a flat formed part, which is formed to the foot (16), which formed part is fixed or fixable to the foot (16) and/or to outer footwear such as a shoe or boot.

10. The ankle orthosis according to claim 9, in which the lower partial section (12) reaches under the sole of the wearer's foot with an angled base section (42).

11. The ankle orthosis according to one of the claims 1 to 10, in which the joint (26) for the movable connection of the lower partial section (12) and the upper partial section (18) is formed by a ball joint.

12. The ankle orthosis according to claim 11, in which the joint (26) or ball joint has an additional degree of freedom by the distance between the two partial sections (12 and 18) perpendicular to their surfaces facing each other being variable within a defined range.

13. The ankle orthosis according to one of the claims 1 to 12, in which there is a defined minimum distance of at least 20 millimeters between the joint (26) and the guide element (28) or its guide slot (62).

## Revendications

1. Orthèse de l'articulation du pied (10) pour limiter une amplitude de mouvement dans l'articulation de la cheville (24) d'un pied humain (16) et/ou pour stabiliser mécaniquement le système ligamentaire dans l'articulation de la cheville (24), laquelle orthèse (10) présente au moins deux sections (12, 18) reliées l'une à l'autre de manière articulée,
- dans laquelle une section inférieure (12) peut être associée à un côté latéral (14) du pied (16) à stabiliser, et
- dans laquelle une partie supérieure (18) peut être associée à un côté latéral (20) du bas de jambe (22) au-dessus de l'articulation de la cheville (24) du pied (16),
- dans laquelle la section supérieure (18) et la section inférieure (12) sont reliées de manière mobile l'une à l'autre au moyen d'une articulation à jeu (26) située à proximité de l'articulation de la cheville (24), et
- dans laquelle un élément de guidage (28) est disposé à distance proximale de l'articulation (26) pour limiter l'angle et/ou l'amplitude de la mobilité et la possibilité de pivotement des sections supérieure et inférieure (18, 12) l'une par rapport à l'autre, lequel élément de guidage (28) restreint ou bien limite respectivement les angles de pivotement maximaux (α, β) aussi bien
(i) dans une dorsiflexion (32) que
(ii) dans une flexion plantaire (34) à des angles critiques définis (α_{Dorsal}, α_{Plantar}), **caractérisée par le fait que** ledit élément de guidage (28) est formé par une coulisse de guidage (62) et par un tenon (58) qui y est guidé, en particulier avec un léger jeu, et peut être déplacé et/ou être placé angulairement.

2. Orthèse de l'articulation du pied (10) selon la revendication 1, dans laquelle ladite coulisse de guidage (62) est formée par une rainure arquée qui guide le tenon (58).

3. Orthèse de l'articulation du pied selon la revendication 1 ou 2, dans laquelle la coulisse de guidage (62) présente un bord extérieur contouré (66), dans laquelle un contour de bord définit et limite l'angle d'attaque (β) entre les sections (12 et 18) reliées l'une à l'autre de manière articulée.

4. Orthèse de l'articulation du pied selon l'une quelconque des revendications 1 à 3, dans laquelle le tenon (58) guidé dans la coulisse de guidage (62) présente à son extrémité une tête élargie (60) qui agit de concert avec le contour de bord correspondant (66) de la coulisse de guidage (62) et assure la limitation des angles d'attaque (β) entre les sections (12 et 18) reliées l'une à l'autre de manière articulée.

5. Orthèse de l'articulation du pied selon l'une quelconque des revendications 1 à 4, dans laquelle le tenon (58) guidé dans la coulisse de guidage (62) présente une base élargie ou un élargissement (68) qui agit de concert avec une face arrière de la coulisse de guidage (62), en particulier avec un contour de bord arrière (74) de la coulisse de guidage (62) et assure la limitation des angles d'attaque (β) entre les sections (12 et 18) reliées l'une à l'autre de manière articulée.

6. Orthèse de l'articulation du pied (10) selon l'une quelconque des revendications 1 à 5, dans laquelle l'élément de guidage (28) limite en sus
(iii) un angle de supination (β_{Sup}) ainsi qu'un angle de pronation (β_{Pro}) dans l'articulation de la cheville (24) en restreignant les angles d'attaque (β) entre les sections supérieure et inférieure (18, 12).

7. Orthèse de l'articulation du pied (10) selon l'une quelconque des revendications 1 à 6, qui sert à la stabilisation mécanique extérieure du système ligamentaire dans l'articulation de la cheville (24), dans laquelle la section inférieure (12) peut être appliquée sur un côté latéral (14) du pied (16) à stabiliser et/ou y être fixé, et/ou dans laquelle la section supérieure (18) peut être appliquée sur un côté latéral (20) du bas de jambe (22) au-dessus de l'articulation de la cheville (24) du pied (16) et/ou y être fixée.

8. Orthèse de l'articulation du pied selon l'une quelconque des revendications 1 à 7, dans laquelle la section supérieure (18) est formée par une pièce moulée plate, dans laquelle la section supérieure (18) peut être fixée au moyen d'un pansement (30) au bas de jambe (22) du porteur.

9. Orthèse de l'articulation du pied selon l'une quelconque des revendications 1 à 8, dans laquelle la section inférieure (12) est formée par une pièce moulée plate et surmoulée sur le pied (16), qui est fixée ou peut être fixée au pied (16) et/ou à un vêtement de dessus destiné au pied, telle qu'une chaussure ou une botte.

10. Orthèse de l'articulation du pied selon la revendication 9, dans laquelle la section inférieure (12) s'engage sous la plante du pied du porteur avec une section de base coudée (42).

11. Orthèse de l'articulation du pied selon l'une quelconque des revendications 1 à 10, dans laquelle l'articulation (26) destinée à relier de manière mobile les sections inférieure et supérieure (18, 12) est formée par une rotule.

12. Orthèse de l'articulation du pied selon la revendication 11, dans laquelle l'articulation (26) ou la rotule présente un degré de liberté supplémentaire par le fait que la distance entre les deux sections (12 et 18) perpendiculaire à leurs surfaces montrant l'une vers l'autre est variable dans une marge définie.

13. Orthèse de l'articulation du pied selon l'une quelconque des revendications 1 à 12, dans laquelle il existe une distance minimale définie d'au moins 20 millimètres entre l'articulation (26) et l'élément de guidage (28) ou bien sa coulisse de guidage (62).
